# FASCICULE DE BREVET EUROPEEN

(11) **EP 2 938 355 B1**
(45) Date de publication et mention de la délivrance du brevet: **10.03.2021**
(21) Numéro de dépôt: 13824600.4
(22) Date de dépôt: 30.12.2013
(51) Int. Cl.: A61K 39/008

(54) **VACCINS MONOVALENTS ET POLYVALENTS CONTRE LEISHMANIA**
MONOVALENTE UND POLYVALENTE IMPFSTOFFE GEGEN LEISHMANIA
MONOVALENT AND MULTIVALENT LEISHMANIA VACCINES

(30) Priorité: 28.12.2012 FR 1203620
(43) Date de publication de la demande: 04.11.2015
(73) Titulaire: VIRBAC S.A., 06516 Carros Cedex (FR); Institut De Recherche Pour Le Développement (IRD), 13002 Marseille 2 (FR)
(72) Inventeur: PAPIEROK, Gérard-Marie, F-83400 Hyères (FR); LEMESRE, Jean-Loup, F-34090 Montpellier (FR); GUEGUEN, Sylvie, F-06410 Biot (FR); BRAS GONCALVES, Rachel, Elise, F-34090 Montpellier (FR); PETITDIDIER-LESIN, Elodie, F-34090 Montpellier (FR)
(74) Mandataire: Macquet, Christophe
(86) Numéro de dépôt international: PCT/FR2013/000373
(87) Numéro de publication internationale: WO 2014/102471

(56) Documents cités:
- WO-A2-2009/153458
- GUERFALI F Z ET AL: "An in silico immunological approach for prediction of CD8+ T cell epitopes of Leishmania major proteins in susceptible BALB/c and resistant C57BL/6 murine models of infection", INFECTION, GENETICS AND EVOLUTION, ELSEVIER, AMSTERDAM, NL, vol. 9, no. 3, 1 mai 2009 (2009-05-01), pages 344-350, XP026145334, ISSN: 1567-1348, DOI: 10.1016/J.MEEGID.2008.02.011 [extrait le 2008-03-04]

## Description

La présente invention concerne des composés peptidiques mono- ou multi-épitopiques destinés à la prévention et au traitement des leishmanioses chez les mammifères et en particulier chez l'Homme, les canidés, les félidés et les équidés. Plus particulièrement, l'invention concerne également des vaccins comprenant lesdits composés peptidiques dans des conditions assurant une immunisation conduisant à une protection efficace de l'Homme ou de l'animal vacciné contre une ou plusieurs leishmanies.

Les grandes endémies parasitaires sont, de très loin, les causes les plus importantes de morbidité et de mortalité non seulement pour l'espèce humaine, mais pour toutes les autres espèces animales tant domestiques que sauvages.

Les leishmanioses sont parmi les plus graves infections parasitaires affectant l'Homme dans le monde. Trois cents cinquante millions d'individus sont exposés dans quatre-vingt-huit pays répartis sur quatre des cinq continents et seize millions d'entre eux sont porteurs du parasite. Elles sont responsables d'un large spectre de manifestations cliniques (cutanée, muco-cutanée et viscérale).

La leishmaniose viscérale (LV) est causée par les parasites du complexe Leishmania donovani (L. *infantum, L. chagasi et L. donovani*) qui sont des parasites intracellulaires du macrophage. Contrairement aux autres espèces de *Leishmania* causant les formes cutanées de la maladie (*L. major, L. brasiliensis,* ...), les leishmanies du complexe *donovani* présentent une capacité à se disséminer à l'ensemble des organes profonds où elles se multiplient. Les patients atteints de LV présentent alors un tableau clinique associant une fièvre modérée mais persistante, une hépatosplénomégalie et une pancytopénie conduisant généralement au décès si aucun traitement spécifique n'est initié suffisamment rapidement.

Même si la LV à L. infantum/chagasi est un problème important en Europe du Sud avec l'extension de la pandémie du SIDA et l'apparition d'un nombre croissant de co-infections *Leishmania*/*HIV,* elle est loin d'être contrôlée et devient très préoccupante en termes de problème de santé publique dans les pays d'Amérique du Sud. Dans d'autres parties du monde, notamment dans les zones d'endémie à L. *donovani,* elle touche environ un cinquième de la population au Soudan et en Inde, où des épidémies meurtrières ont fait des centaines de milliers de victimes ces dernières décennies. De plus, la situation devient critique en Inde avec l'émergence de souches résistantes aux dérivés pentavalents de l'antimoine (médicaments de première intention).

L'incidence annuelle des leishmanioses humaines est estimée entre 2 et 2,5 millions de nouveaux cas, parmi lesquels plus de 500 000 sont atteints de leishmaniose viscérale et 1,5 à 2 millions de leishmanioses cutanées dont certaines sont très défigurantes et mutilantes comme les leishmanioses muco-cutanées (LMC). Ceci a pour conséquence une morbidité globale supérieure à deux millions de DALYs (pour Disability Adjusted Life Years) et le décès de près de soixante mille personnes chaque année. Ces maladies constituent encore aujourd'hui un véritable problème de santé publique en Amérique latine, en Asie, en Afrique et dans le sud de l'Europe. Leurs conséquences sur de très nombreuses régions du monde en font un handicap majeur au développement des pays les plus pauvres. De plus, l'effet global des changements environnementaux (anthropiques et climatiques) conduit soit à une augmentation de l'incidence (maladies « ré-émergentes » et incontrôlées dans de nombreuses régions du monde), soit à une extension géographique (maladies « émergentes » notamment dans le nord de l'Europe et en Amérique du nord) de ces maladies parasitaires. Elles restent cependant délaissées par la recherche médicale, les financeurs, l'opinion publique et les instances politiques des pays gravement touchés.

La leishmaniose viscérale touche également la population canine, qui constitue un réservoir de parasites approvisionnant de manière continue le cycle de transmission. Elle est due à *L. infantum*/*chagasi,* espèce responsable d'une zoonose des canidés, domestiques et sauvages largement répandue dans le monde. Elle affecte des millions de chiens en Europe, Asie, Afrique du Nord et Amérique du Sud. Les chiens symptomatiques mais aussi asymptomatiques constituent une source de parasites pour la transmission de la LV chez l'Homme.

Les mesures de prévention contre les leishmanioses sont nombreuses. Pendant longtemps elles ont été fondées sur la lutte contre l'insecte vecteur et l'objectif principal visait à contrôler leur multiplication par l'utilisation d'insecticides et à éviter le contact entre le vecteur (phlébotome) et leurs hôtes (chien, Homme, ...). Le contrôle des leishmanioses, une fois diagnostiquées, passe aussi par les traitements chimiothérapeutiques. Ces derniers sont malheureusement mis en péril par un arsenal thérapeutique très limité, des traitements longs, toxiques et onéreux s'accompagnant de nombreux cas de rechute et par l'émergence des phénomènes de chimiorésistance. En l'absence de traitements efficaces et devant l'ampleur du problème, il devient donc nécessaire de mettre à la disposition des populations les plus gravement touchées des moyens de prévention applicables à grande échelle, dont le plus adapté serait la vaccination.

Plusieurs arguments sont en faveur de la faisabilité d'un vaccin chez l'Homme. Le fait qu'un individu vivant en zone d'endémie puisse développer une forte résistance à la leishmaniose cutanée, suite à l'injection volontaire d'une petite quantité de parasites vivants a très vite montré que la vaccination contre ces parasitoses était possible chez l'Homme. Bien qu'extrêmement efficace (plus de 80% de protection), cette pratique, utilisant des promastigotes de *L. major,* encore appelée « leishmanisation », a longtemps été utilisée, notamment au moyen Orient. Elle a par la suite été abandonnée pour des raisons éthiques et sécuritaires. En effet, elle provoquait des formes graves de leishmaniose chez 2 à 3 % des sujets (Ferrua et al, Vaccine 2006*).* De plus, il est maintenant bien établi que, suite à l'infection par des leishmanies, la grande majorité des individus immunocompétents développe des infections subcliniques ou asymptomatiques plutôt qu'une forme sévère de leishmaniose (symptomatique), et de ce fait, acquiert une immunité robuste et durable à la réinfection. Il en est de même chez les individus ayant contrôlé une leishmaniose cutanée ou après une leishmaniose viscérale guérie.

Malgré cela, chez l'Homme, aucun vaccin n'est actuellement disponible contre les leishmanioses. En fait, seule une approche pragmatique utilisant des parasites entiers tués a été rendue possible à ce jour. Historiquement, les vaccins de première génération, basés sur l'utilisation de parasites entiers atténués ou morts (notamment autoclavés) combinés ou non à des adjuvants, avaient pour but de protéger principalement contre les leishmanioses cutanées en essayant de reproduire les niveaux de protection obtenus avec les parasites vivants.

Cependant, les nombreux essais cliniques menés sur des milliers d'individus en Amérique du sud mais aussi en Iran ont donné des résultats plus que mitigés, voire décevants (Modabber, International Journal of Antimicrobial Agents, 2010 *;* Evans et Kedzierski, Journal of Tropical Medicine, 2012*).*

Les infections expérimentales chez la souris, notamment la souche murine BALB/c susceptible à beaucoup d'espèces de leishmanies, ont été et sont encore très utilisées pour évaluer l'efficacité d'un candidat vaccin notamment à l'encontre d'une leishmaniose viscérale. De nombreux vaccins à sous-unités constitués de protéines natives purifiées (vaccin de première génération) ou recombinantes (vaccin de seconde génération) ont ainsi été testés (Goto et al, clinical and Vaccine Immunology, 2011). Certains candidats vaccins ont conféré un bon niveau de protection à l'encontre d'une infection expérimentale chez la souris. Les protections pouvaient atteindre jusqu'à 81% selon l'antigène et l'adjuvant. Plus récemment, les vaccins ADN, dit de troisième génération, plus faciles à produire, plus stables et plus immunogéniques ont été développés avec les séquences nucléotidiques codant pour les protéines utilisées dans les vaccins de seconde génération (Gurunathan et al, Nature Med., 1998*;* Dumonteil et al, Vaccine, 2003*;* Rafati et al, Vaccine, 2005 *;* Rodriguez-Cortes et al, Vaccine, 2007). Enfin d'autres pistes vaccinales ont été explorées avec succès chez la souris comme les vaccins à base d'antigènes présents dans la salive du phlébotome (Morris et al, J. Immunol., 2001*;* Valenzuela et al, J. Exp. Biol., 2004*),* qui ont permis d'obtenir une relative résistance à l'infection expérimentale à *Leishmania major.*

A ce jour, peu de candidats vaccins ont dépassé le stade expérimental (modèles souris ou hamsters) en raison de la difficulté à développer un modèle animal approprié reproduisant les caractéristiques de la maladie humaine et à mettre en œuvre des essais cliniques chez des hôtes naturels de l'infection qui nécessitent des cohortes importantes, des infrastructures lourdes et qui peuvent prendre des années avant de donner un résultat définitif. De plus, il est difficile d'extrapoler directement des résultats obtenus chez la souris à des hôtes naturels de l'infection, tels que le chien ou l'Homme. Enfin, les réponses immunitaires protectrices contrôlant une infection d'épreuve peuvent ne pas refléter celles requises pour prévenir les leishmanioses en zones endémiques.

Le document WO 2009/153458 A2 divulgue un peptide de synthèse non natif cyclisé par pont disulfure et un complexe de peptides de synthèse non natifs destiné à induire et à caractériser la prévention et/ou le traitement d'affections chez les mammifères dont l'immunité protectrice dépend de la stimulation des lymphocytes de type Th1.

Deux candidats vaccinaux sont en cours de développement clinique :
- Le Leish 111 f MPL SE du Infections Disease Research Institute (IRDI) à Seattle est un vaccin chimérique appelé LEISH-F1 constitué de 3 protéines recombinantes en fusion (TSA-LmSTI1-LeIF) formulées avec du lipide monophosphorylé et du squalène dans une émulsion stable (MPL-SE). Toutefois, si la protection contre la leishmaniose cutanée semble en bonne voie, une étude menée en Italie dans une station expérimentale montre clairement que Leish-111 f MPL-SE ne protège pas des chiens exposés à une infection naturelle à L. *infantum* (Gradoni et al, Veterinary Research, 2005 ; Gradoni, Veterinary Research, 2006*).* Dans une étude plus récente réalisée au Brésil, ce vaccin s'est révélé efficace en immunothérapie pour les cas légers de LV canine mais sans effet chez des chiens gravement malades *(*Trigo et al, Clinical and Vaccine Immunology, 2010).
- Le HASP + KMP-11 de l'Université de York (Grande Bretagne) sur lequel les premiers essais cliniques devraient démarrer fin 2012.

Chez le Chien, il existe à ce jour une solution efficace, CaniLeish®, qui est un produit composé d'antigènes d'excrétion sécrétion (AES) de promastigotes de L. *infantum* et commercialisé en Europe depuis 2011. La leishmaniose canine voit ainsi ses mesures préventives profondément modifiées et complétées.

La vaccination contrôle non seulement le développement de la maladie mais diminue aussi significativement la charge parasitaire chez le chien et contribue ainsi à l'interruption du cycle de transmission de la LV chez le phlébotome (vecteur de la maladie), le chien et l'Homme.

Toutefois les rendements de production des AES ne sont pas suffisants pour permettre une production à l'échelle industrielle d'un vaccin humain. En effet, la production des AES est assez complexe et induit un produit à prix trop élevé, adapté à un marché canin assez réduit.

Ainsi, une des difficultés principales dans le développement d'un candidat vaccinal réside dans le fait que le produit final doit être reproductible, thermostable et facile à produire à bas prix dans les zones d'endémie, avec des rendements de production rendant possible son utilisation à grande échelle. En effet, les résultats d'une étude récente visant à évaluer la rentabilité économique d'un vaccin contre la leishmaniose viscérale dans l'état du Bihar (Inde) comparativement à la chimiothérapie, appuient fortement la poursuite du développement d'un vaccin contre la LV, même si son coût s'avérerait relativement élevé (Lee et al, Am. J. Trop. Med. Hyg., 2012*).*

Compte tenu de ce qui précède, il existe à ce jour un besoin urgent d'investir dans de nouvelles pistes proposant une alternative plus efficace aux vaccins existants, afin de permettre la vaccination prophylactique et/ou thérapeutique de patients contre les leishmanies, notamment les patients résistants aux thérapies existantes.

Le Demandeur a mis en évidence de nouveaux épitopes présentant un fort pouvoir immunogène contre les leishmanioses, permettant le développement de vaccins efficaces et financièrement accessibles.

Ainsi, la solution au problème posé a pour premier objet un épitope présentant une séquence choisie parmi :
SEQ ID N°1 : T-P-E-Q-R-T-N-T-L (épitope HLA-B07) ;
SEQ ID N°2 : E-L-G-K-K-W-I-G (épitope HLA-B08) ;
SEQ ID N°3 : T-L-P-E-M-P-V-G-V (épitope HLA-A02) ;
SEQ ID N°4 : P-E-M-P-A-G-V-D-Y (épitope HLA-A01) ;
SEQ ID N°5 : A-R-G-R-E-G-Y-F-L (épitope HLA-B27) ;
SEQ ID N°6 : A-R-G-A-R-G-R-E-G-Y (épitope HLA-B44) ;
SEQ ID N°7 : A-R-G-A-R-G-R-E-G (épitope HLA-B27) ;
SEQ ID N°8 : E-G-Y-F-V-T-D-E-K (épitope HLA-A03) ; 
SEQ ID N°9 : T-N-T-L-A-V-L-Q-A-F-G-R-A-I-P-E-L-G-K-
K-W ;
 
SEQ ID N°10 : E-G-Y-F-V-T-D-E-K-T-G-L-V-Y-R-D-G-G-
V-A-A-A-S-S-G;
SEQ ID N°15: X₁-X₂-P-E-M-P-X₃-G-V-X₄-X₅
   avec
   X₁ = T ou aucun acide aminé
   X₂ = L ou aucun acide aminé
   X₃ = A ou V
   X₄ = D ou aucun acide aminé
   X₅ = Y ou aucun acide aminé ; ou SEQ ID N°16 : A-R-X₆-X₇-X₈-G-R-E-G-X₉-X₁₀-X₁₁
   avec
   X₆ = G ou aucun acide aminé
   X₇ = A ou aucun acide aminé
   X₈ = R ou aucun acide aminé
   X₉ = Y ou aucun acide aminé
   X₁₀ = F ou aucun acide aminé
   X₁₁ = L ou aucun acide aminé

Afin de répondre au besoin d'un vaccin assurant une bonne couverture de la population mondiale et protégeant contre les principales espèces de leishmaniose, le Demandeur a considéré l'importance de proposer une stratégie vaccinale innovante basée sur des fragments antigéniques/peptidiques capables d'activer, de façon durable, l'immunité cellulaire spécifique dirigée contre ces parasites. La stratégie vaccinale peptidique du Demandeur s'appuie sur l'identification et la sélection de peptides immunodominants portés par la séquence d'une protéine de virulence caractérisée comme l'immunogène majeur des antigènes excrétés-secrétés par les leishmanies, la protéine PSA (pour Promastigote Surface Antigen) soluble (principe actif principal du vaccin CaniLeish®), commune et très conservée au sein des espèces de leishmanies, responsables des différentes infections humaines.

La vaccination peptidique repose sur les bases moléculaires et cellulaires de la reconnaissance de l'antigène par les cellules T. La mise en place de l'immunité spécifique dépend pour une large mesure de la dégradation et de l'association des fragments antigéniques, peptides, aux molécules du Complexe Majeur d'Histocompatibilité (CMH ; HLA, de l'anglais human leukocyte antigen, pour l'Homme). Cette association est rendue spécifique d'une molécule HLA particulière par des résidus acides aminés constituant les motifs d'ancrage du peptide. Les complexes ainsi formés sont reconnus par les lymphocytes T par l'intermédiaire d'un récepteur membranaire (TcR) et nécessite une interaction spécifique avec certains acides aminés de l'épitope T. Les épitopes T sont des ligands des molécules HLA avec des affinités fortes ou modérées. Ils sont présentés aux lymphocytes T CD8+ (cytotoxiques) ou CD4+ (auxiliaires) par les molécules HLA respectivement de classe I ou de classe II.

La formation de ces complexes tri-moléculaires (TcR/HLA/peptide) est le prérequis à l'activation et à l'expansion des cellules T spécifiques et donc à l'induction d'une réponse immunitaire protectrice lors d'une infection.

L'intérêt de tels peptides est multiple :
(i) ils présentent une grande innocuité puisqu'ils évitent tout risque infectieux lié à l'utilisation d'agent pathogène ou d'agent infectieux,
(ii) ils sont chimiquement bien définis et répondent ainsi aux exigences pharmaceutiques (notamment pureté, reproductibilité),
(iii) ils facilitent le monitoring de la réponse immunitaire induite (recherche de lymphocytes T cytotoxiques (CTL) dirigés contre un épitope T défini), et
(iv) leur séquence peut être modifiée afin de les rendre plus immunogènes.

De tels peptides répondent de façon remarquable à l'ensemble des conditions citées précédemment : vaccin reproductible, thermostable facilitant sa conservation et son transport, polyvalent, facile à produire à bas prix dans les zones d'endémie, rendant possible son utilisation à grande échelle.

Selon l'invention, on entend par épitope, composé peptidique ou peptide, tout épitope, composé peptidique ou peptide défini par sa séquence, ainsi que ses dérivés analogues, mutéines et homologues.

Les épitopes étant des composés peptidiques présentant environ 8 à 15 acides aminés, on utilise donc indifféremment le terme « composé peptidique » pour désigner un épitope ou un composé peptidique.

Selon l'invention, on entend par « dérivé analogue » ou « dérivés mutéines » d'un composé peptidique, les dérivés biologiquement actifs des molécules de référence qui présentent l'activité souhaitée, à savoir la capacité à stimuler une réponse immunitaire à médiation cellulaire.

De façon générale, le terme « dérivé analogue », se réfère à des composés ayant une séquence et une structure polypeptidique présentant une ou plusieurs additions, substitutions et/ou délétions d'acide aminé, par rapport aux composés peptidiques définis ci-dessus, dans la mesure où les modifications ne détruisent pas l'activité immunogène.

On entend par le terme « dérivé mutéine », les peptides présentant un ou plusieurs éléments imitant le peptide.

Des procédés de préparation d'analogues et de mutéines polypeptidiques classiques sont connus de l'homme du métier.

Selon l'invention, les analogues particulièrement préférés incluent les substitutions conservatrices, c'est-à-dire les substitutions ou remplacement sans conséquences sur la fonction et la structure finale de la protéine.

A titre d'exemples de telles substitutions, on peut citer les substitutions intervenant dans :
- la famille d'acides aminés composée de l'aspartate et du glutamate ;
- la famille d'acides aminés basiques composée de la lysine, de l'arginine et de l'histidine ;
- la famille d'acides aminés non polaires composée de l'alanine, de la leucine, de l'isoleucine, de la proline, de la phénylalanine, de la méthionine et du tryptophane ; et
- la famille des acides aminés non chargés polaires tels que la glycine, l'asparagine, la glutamine, la cystéine, la sérine, la thréonine et la tyrosine.

Par exemple, compte tenu du fait que, d'un point de vue stérique et physico-chimique, la valine est proche de l'alanine; la substitution de l'une par l'autre ne perturbe généralement pas le fonctionnement de la protéine.

L'homme du métier pourra aisément déterminer les régions des composés peptidiques d'intérêt pouvant tolérer un changement par des techniques bien connues.

Pour cela, il pourra par exemple utiliser des matrices de similarité, M, qui recensent l'ensemble des scores *M(a,b)* obtenus lorsqu'on substitue l'acide aminé *a* par l'acide b dans une séquence peptidique donnée. Il existe plusieurs de ces matrices de taille 20 x 20, pour les 20 acides aminés, avec des modes de construction différents.
Parmi les plus classiques, on peut citer les matrices de Dayhoff, appelées PAM pour « *probability of acceptable mutations »,* basées sur des distances évolutives entre espèces et les matrices de Henikoff, appelées BLOSUM, basées sur le contenu en information des substitutions.

Dans chaque famille, il existe plusieurs séries de matrices, de stringence variable, et donc plus ou moins tolérantes aux substitutions d'acides aminés.
Un diagramme de Venn peut également être utilisé pour mettre en évidence les relations existant entre les 20 acides aminés d'origine naturelle en fonction d'une sélection de propriétés physico-chimiques considérées comme importantes dans la détermination de la structure des protéines.

Selon l'invention, on entend par « dérivé homologue », des composés peptidiques présentant un certain pourcentage d'identité peptidique. Le terme « identité » signifie que les acides aminés de deux séquences peptidiques comparées correspondent exactement. Le pourcentage d'identité se détermine par une comparaison directe des séquences entre deux composés peptidiques en alignant lesdites séquences et en comptant le nombre exact de mésappariement entre les deux séquences alignées. Ensuite, on divise par la longueur de la séquence la plus courte et on multiplie le résultat par cent. Le pourcentage d'identité peut également être déterminé à l'aide de programmes d'ordinateurs bien connus de l'homme du métier tels que Dotlet, produit par la société Dotplot, Clustal X et W tels que décrits dans la publication Thompson JD, Gibson TJ, Plewniak F, Jeanmougin F, Higgins DG: The CLUSTAL_X windows interface: flexible stratégies for multiple sequence alignment aided by quality analysis tools. Nucleic Acids Res 1997, 25 (24):4876-4882 ou encore Morgenstern B tel que décrit dans la publication DIALIGN 2: improvement of the segment-tosegment approach to multiple sequence alignment. Bioinformatics 1999, 15(3):211-218.
La base BLOCKS (Henikoff et Henikoff,1991) donne sous forme d'alignements multiples sans insertion-délétion (ou blocs) les sous- séquences de Swissprot qui correspondent à des régions conservées.

Ainsi, selon l'invention, deux séquences peptidiques sont dites « sensiblement homologues » l'une par rapport à l'autre, dès lors qu'elles présentent au moins 50%, de préférence au moins 75%, de préférence encore au moins 85%, de préférence encore au moins 90% et d'avantage préféré au moins 95% ou plus d'identité de séquence sur une longueur définie des molécules peptidiques.

En outre, de façon avantageuse, les épitopes ou composés peptidiques objet de l'invention sont reliés à des porteurs permettant de les rendre d'avantage immunogènes. A titre d'exemple non limitatif de porteur, on peut citer les molécules KLH pour Keyhole Limpet Hemocyanin, les lipopeptides de type palmitoyl, ou leurs dérivés.

Les modifications les plus courantes de protéines par des lipides sont : l'isoprénylation, la N-myristoylation, la palmitoylation (ou S-acylation) et la glypiation.

L'isoprenylation et la N-myristoylation sont des modifications co-traductionnelles ou immédiatement post-traductionnelles et le groupement qui est attaché le reste jusqu'à la dégradation de la protéine.

La palmitoylation est post-traductionnelle. Cette modification est réversible et plus rapide que le "turn-over" de la dégradation des protéines : elle peut donc être régulée. La glypiation est co- et post-traductionnelle.

Les peptides selon l'invention sont préférentiellement des peptides palmitoylés car ces dérivés interagissent avec les composants lipidiques de la membrane des cellules cibles, que sont par exemple les macrophages, les cellules dendritiques ou encore les neutrophiles, favorisent leur pénétration et les véhiculent à l'intérieur de celles-ci pour les présenter au système immunitaire.

On peut également citer les peptides glycosylés comme, par exemple, les conjugués mannosylés et les construction MAPs, pour Multiple Antigenic Peptides.

Les épitopes objet de l'invention peuvent en outre contenir un ou plusieurs groupements protecteurs.

En effet, de façon à améliorer la résistance à la dégradation, il peut être nécessaire d'utiliser une forme protégée du peptide selon l'invention. La forme de protection doit évidemment être une forme biologiquement compatible et doit être compatible avec une utilisation dans le domaine pharmaceutique De nombreuses formes de protection biologiquement compatibles peuvent être envisagées, elles sont bien connues de l'homme du métier comme, par exemple, l'acylation ou l'acétylation de l'extrémité amino-terminale, ou l'amidation ou l'estérification de l'extrémité carboxy-terminale. Ainsi, l'invention concerne également un épitope tel que défini précédemment, caractérisé par le fait qu'il se présente sous forme protégée ou non. On peut utiliser une protection basée sur une substitution sur l'extrémité amino-terminale par un groupement acétyle, un groupement benzoyle, un groupement tosyle ou un groupement benzyloxycarbonyle. De préférence, on utilise une protection basée sur l'amidation de la fonction hydroxyle de l'extrémité carboxy-terminale par un groupement NYY avec Y représentant une chaîne alkyle de C1 à C4, ou l'estérification par un groupement alkyle.

Il est également possible de protéger les deux extrémités du composé peptidique. Les dérivés de peptides concernent aussi les acides aminés et les peptides reliés entre eux par une liaison pseudo-peptidique. On entend par liaison pseudo-peptidique, tous les types de liaisons susceptibles de remplacer les liaisons peptidiques classiques. Dans le domaine des acides aminés, la géométrie des molécules est telle qu'elles peuvent théoriquement se présenter sous la forme d'isomères optiques différents. Il existe, en effet, une conformation moléculaire de l'acide aminé (aa) telle qu'elle dévie à droite le plan de polarisation de la lumière (conformation dextrogyre ou D-aa), et une conformation moléculaire de l'acide aminé (aa) telle qu'elle dévie à gauche le plan de polarisation de la lumière (conformation lévogyre ou L-aa). Les acides aminés naturels sont toujours de conformation lévogyre, en conséquence, un peptide d'origine naturelle ne sera constitué que d'acides aminés de type L-aa. Cependant, la synthèse chimique en laboratoire permet de préparer des acides aminés ayant les deux conformations possibles.

A partir de ce matériel de base, il est ainsi possible d'incorporer lors de la synthèse de peptide aussi bien des acides aminés sous forme d'isomères optiques dextrogyre ou lévogyre. Ainsi, les acides aminés constituant le peptide selon l'invention peuvent être sous configuration L-, D- ou DL-.

Les épitopes et composés peptidiques selon l'invention peuvent être obtenus soit par synthèse chimique classique (en phase solide ou en phase homogène liquide), soit par synthèse enzymatique, à partir d'acides aminés constitutifs ou de leurs dérivés. Les épitopes et composés peptidiques selon l'invention peuvent également être obtenus par fermentation d'une souche de bactéries modifiées ou non, par génie génétique, ou encore par extraction de protéines d'origine animale ou végétale, préférentiellement d'origine végétale, suivie d'une hydrolyse contrôlée qui libère des fragments peptidiques correspondant totalement ou partiellement aux épitopes et composés peptidiques selon l'invention.

Comme cela est montré aux exemples 1 à 4, le Demandeur a pu mettre en évidence que les différents épitopes selon l'invention sont des séquences consensus communes aux principales espèces de leishmanies et présentent une forte et moyenne affinité pour l'ensemble des molécules du CMH (Complexe Majeur d'Histocompatibilité) des mammifères, et plus particulièrement pour l'ensemble des molécules du HLA (HLA pour antigènes des leucocytes humains), majoritairement représentées dans les populations humaines les plus gravement touchées par ces affections.

En effet, dans le contexte d'une stratégie vaccinale des populations humaines exposées aux infections leishmaniennes et afin de répondre au besoin d'un vaccin assurant une bonne couverture de la population mondiale et protégeant contre les leishmanioses, il est important d'utiliser des fragments antigéniques/peptidiques capables d'activer, de façon durable, l'immunité cellulaire spécifique dirigée contre le parasite.

Pour assurer une bonne couverture de la population mondiale et au regard de la grande variabilité du phénotype HLA (complexe majeur d'histocompatibilité humain) entre les individus, les fragments antigéniques immunogènes (peptides) de longueur suffisante doivent contenir une série d'épitopes aptes à être présentés par plusieurs types de molécules HLA de classe I et II.

Les molécules HLA sont hautement polymorphes. En effet, il existe plus de 2500 protéines HLA de classe I (HLA-I) et plus de 1000 protéines HLA de classe II (HLA-II). Cependant, certaines de ces molécules HLA, proches en séquence et en conformation spatiale, peuvent présenter des épitopes communs aux cellules T. Le regroupement de plusieurs milliers de molécules HLA est aujourd'hui décrit en un peu plus d'une vingtaine de catégories, appelées « supertypes HLA » présentant des épitopes très conservés pour chaque supertype.

Au développement de vaccins peptidiques, s'ajoute l'approche multiépitopique ou polyépitopique (peptide contenant plusieurs épitopes). Cette approche multiépitopique est avantageuse pour mettre point un vaccin destiné à l'ensemble de la population mondiale. En effet, pour assurer une bonne couverture de la population mondiale et au regard de la grande variabilité du phénotype HLA entre les individus, les fragments antigéniques immunogènes (peptides) de longueur suffisante doivent contenir une série d'épitopes aptes à être présentés par plusieurs supertypes de molécules HLA-I et -II.

Aussi, les épitopes objet de l'invention présentent un fort pouvoir immunogène.

Les épitopes T sont des séquences antigéniques que reconnaissent les lymphocytes T. Par exemple chez l'Homme, les épitopes T sont issus de la dégradation des antigènes par les cellules présentatrices et sont présentés aux lymphocytes T CD8+ (cytotoxiques) ou CD4+ (auxiliaires) par les molécules HLA respectivement de classe I ou de classe II. Les épitopes T sont donc nécessairement des ligands des molécules HLA et font effectivement partie des peptides qui se lient aux molécules HLA avec des affinités fortes ou modérées.

Les cellules exprimant le complexe majeur d'histocompatibilité de classe II (CMH2) peuvent aussi présenter des antigènes microbiens via CD1 aux lymphocytes T gamma-delta.

Les capacités de présentation dépendent de nombreuses variables. Elles varient d'un individu à l'autre en raison du polymorphisme du complexe majeur d'histocompatibilité (CMH).

Ainsi, la consanguinité, en diminuant le nombre de CMH différents exprimés par un individu, diminue ses capacités immunes. Elles diffèrent aussi selon les modalités d'exposition à l'antigène (dose et voie d'administration), en raison des variations dans les capacités de présentation des différents types de cellules présentatrices. Par exemple, les cellules impliquées dans la présentation seront différentes par voie cutanée ou digestive.

Enfin, la gamme peptidique produite par un antigène donné sera différente selon la cellule présentatrice (modalités de clivage), et selon l'espèce et l'individu (allèle du CMH).

Les composés peptidiques selon l'invention ont été sélectionnés et conçus en vue d'assurer la couverture vaccinale et thérapeutique des populations les plus gravement touchées par les principales espèces pathogènes de leishmanies. Ils sont destinés à induire et à caractériser la prévention ou le traitement d'affections chez les mammifères dont l'immunité protectrice dépend de la stimulation des lymphocytes de type Th1 et des cellules T cytotoxiques, caractéristique d'un état d'hyperstimulation de type retardée.

De nombreuses situations pathologiques sont associées à un état immunitaire de type Th1 ou Th2.

Pour exemple, l'exacerbation de la voie Th2, correspondant à un état d'hyperstimulation immédiate, induit certaines affections comme les dermites atopiques canines, les allergies et l'asthme.

L'obtention d'une guérison pour ces affections est effectuée par des immunostimulants permettant le passage d'un état immunitaire de type Th2 à un état de type Th1.

D'autre part, cet état immunitaire Th1 est en pleine corrélation avec un état de résistance aux pathogènes intracellulaires tels que *Leishmania, Trypanosoma, Candida, Mycobacterium et Listeria.*

Concernant l'immunopathologie de l'asthme, la littérature au cours de la dernière décennie a désigné le lymphocyte T spécifique de l'allergène comme le chef d'orchestre de la réaction immunoallergique (Magnan. A et al. "Cytokines, from atopy to athsma: the Th2 dogma revisited" Cell Mol Biol, 2001, 47, 679-687). Parmi les lymphocytes T, la sous population Th2 productrice d'IL-4 semble au premier plan et nécessaire à l'induction de cette réaction. Il est dès lors opportun que des stratégies soient établies pour cibler spécifiquement les lymphocytes et en particulier les Th2.

Ce type de stratégie peut être également suivie pour les leishmanioses en polarisant les réponses à médiation cellulaire vers un état immunitaire de type Th1 et en favorisant les réponses cellulaires cytotoxiques, permettant de s'opposer et de contrôler le processus infectieux.

Ainsi que cela est décrit ci-dessus, une autre difficulté principale dans le développement d'un candidat vaccinal réside dans le fait qu'il doit idéalement être efficace contre plusieurs espèces de leishmanies et notamment contre les formes cliniques les plus sévères (viscérale et cutanée) et chez différents hôtes naturels de l'infection (Homme, chien).

Ainsi, l'invention a pour second objet un composé peptidique comprenant 1, 2, 3 ou 4 des épitopes de séquences SEQ ID N° 1 à 10 tels que décrits ci-dessus, éventuellement séparés par un espaceur peptidique comprenant 1 à 8 acides aminés.

A titre d'exemple non limitatif d'espaceur peptidique, on peut citer les motifs T-V, V, Y, L (Lee Y. Et al., Biomed Microdevices, 2010, 12: 207-222 .

De préférence, les espaceurs peptidiques utilisés sont Y ou T-V, ou leurs analogues.

A titre de composés peptidiques comprenant 2 des épitopes de séquence SEQ ID N°1 à 10 tels que décrits ci-dessus, on peut citer:
SEQ ID N°11 : T-P-E-Q-R-T-N-T-L-T-V-E-L-G-K-K-W-I-G ; ou
SEQ ID N°12 : T-L-P-E-M-P-V-G-V-P-E-M-P-A-G-V-D-Y ; ou

SEQ ID N°13 : A-R-G-R-E-G-Y-F-L-A-R-G-A-R-G-R-E-G-
Y-E-G-Y-F-V-T-D-E-K ;

A titre de composés peptidiques comprenant 3 des épitopes de séquence SEQ ID N°1 à 10 tels que décrits ci-dessus, on peut citer:
A titre de composés peptidiques comprenant 4 des épitopes de séquence SEQ ID N°1 à 10 tels que décrits ci-dessus, on peut citer:
L'invention sera mieux comprise à la lecture de la description non limitative qui suit, rédigée au regard des dessins annexés, dans lesquels :

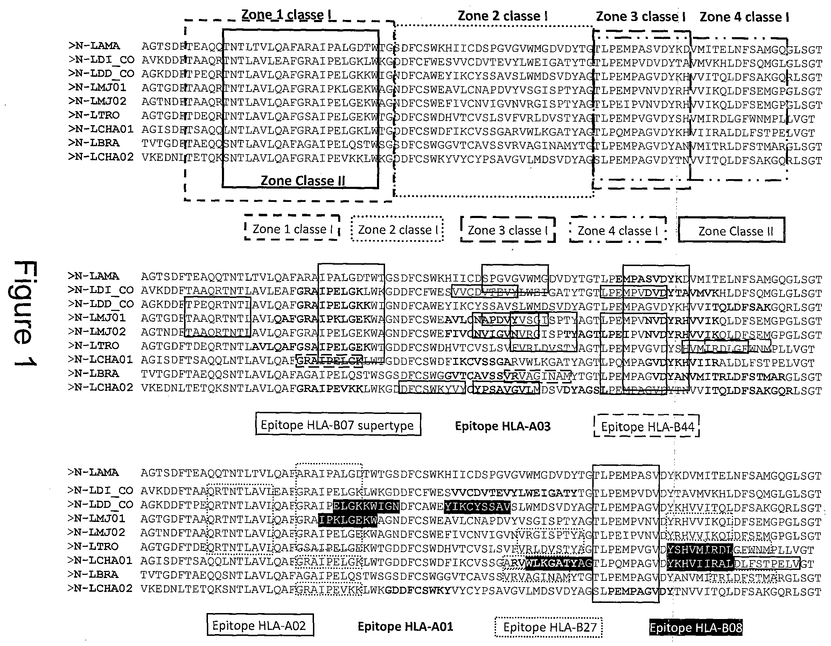

- la figure 1 représente la localisation des épitopes HLA-I et HLA-II sur les séquences Nter-PSA des principales espèces de leishmanies.

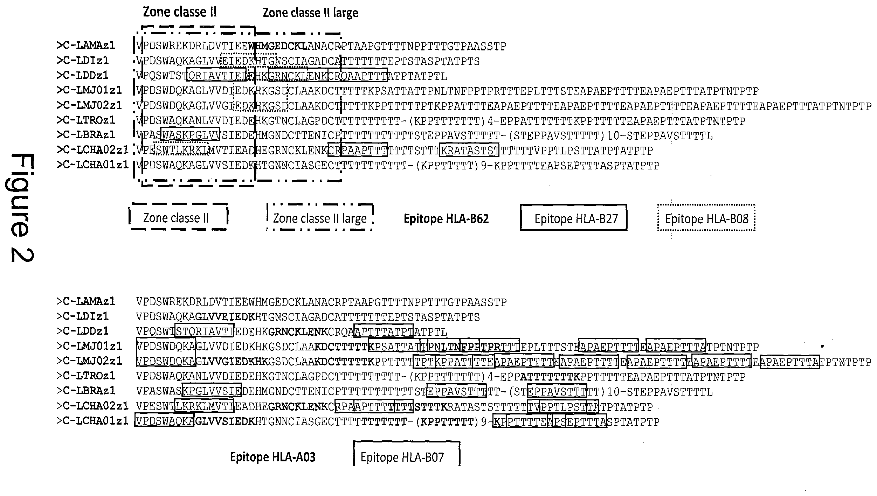

- la figure 2 représente la localisation des épitopes HLA-I et HLA-II sur les séquences Cter-PSA des principales espèces de leishmanies, en particulier sur le début de la séquence Cter-PSA appelée « zone 1 ».

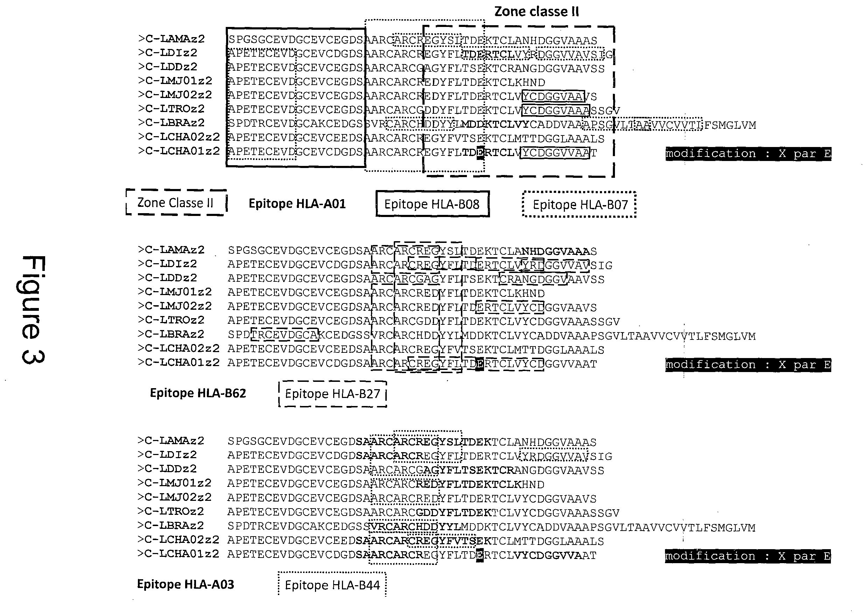

- la figure 3 représente la localisation des épitopes HLA-I et HLA-II sur les séquences Cter-PSA des principales espèces de leishmanies, en particulier sur la seconde partie de la séquence Cter-PSA appelée « zone 2 ».
   Ces figures 1 à 3 représentent des cartes de prédiction *in silico* des zones riches en épitopes à forte et moyenne affinités pour les molécules HLA classe I et HLA classe II sur les séquences amino (Nter)- et carboxy (Cter)-terminales des PSA.
- la figure 4 représente les composés peptidiques SEQ ID N°11 à 14 ainsi que leur découpage en épitopes et la position d'un espaceur entre les épitopes SEQ ID N° 1 et SEQ ID N°2 du composé peptidique SEQ ID N°12.
- la figure 5 représente le résultat d'un algorithme de prédiction des sites de clivage par le protéasome de différents composés peptidiques issus de la partie amino-terminale de différentes PSA.
- la figure 6 représente le schéma d'injection utilisé dans l'étude de l'état immunitaire des chiens avant et après vaccination avec les composés selon l'invention.

D'autres exemples de composés petidiques selon l'invention sont décrits aux exemples 3 à 8.

De préférence, les composés peptidiques selon l'invention sont choisis parmi: 
SEQ ID N°9 : T-N-T-L-A-V-L-Q-A-F-G-R-A-I-P-E-L-G-K-
K-W ; ou
 
SEQ ID N°10 : E-G-Y-F-V-T-D-E-K-T-G-L-V-Y-R-D-G-G-
V-A-A-A-S-S-G ; ou
 SEQ ID N°11 : T-P-E-Q-R-T-N-T-L-T-V-E-L-G-K-K-W-I-G ; ou
SEQ ID N°12 : T-L-P-E-M-P-V-G-V-P-E-M-P-A-G-V-D-Y ; ou

SEQ ID N°13 : A-R-G-R-E-G-Y-F-L-A-R-G-A-R-G-R-E-G-
Y-E-G-Y-F-V-T-D-E-K ;

Comme indiqué précédemment, selon l'invention, les composés peptidiques SEQ ID N°9 à 13 incluent leurs dérivés analogues, mutéines et homologues.

Parmi les dérivés envisageables des composés peptidiques SEQ ID N°9 à 13 ci-dessus, on choisira préférentiellement les dérivés peptidiques des composés ci-dessus comportant au moins cinq acides aminés contigus.

Un troisième objet de l'invention concerne une composition comme produit pharmaceutique, à usage vétérinaire ou humain comprenant au moins:
- un épitope de séquence choisi parmi les séquences SEQ ID N°1 à 10; ou
- un composé peptidique comprenant 1, 2, 3 ou 4 des épitopes de séquence SEQ ID N°1 à 10, éventuellement séparés par un espaceur tel que défini ci-dessus; ou
- un composé peptidique de séquence choisie parmi SEQ ID N°11 à 13.

L'invention concerne également une telle composition pour son utilisation dans la vaccination prophylactique et thérapeutique dirigée contre une ou plusieurs des leishmanies tels que *Leishmania donovani, Leishmania infantum, Leishmania chagasi, Leishmania mexicana, Leishmania amazonensis, Leishmania venezuelensis, Leishmania tropica, Leishmania major, Leishmania aethiopica, Leishmania (Viannia) braziliensis, Leishmania (Viannia) guyanensis, Leishmania (Viannia) panamensis, Leishmania (Viannia) peruviana.*

Avantageusement, la composition selon l'invention est apte à être utilisée dans la vaccination prophylactique et thérapeutique dirigée contre au moins 3, de préférence au moins 7, de préférence encore au moins 10 et d'avantage préféré contre l'ensemble des leishmanies listées ci-dessus.

De préférence, la composition selon l'invention est administrée par voie sous-cutanée, intradermique, intramusculaire, intraveineuse, parentérale ou orale.

Un objet additionnel de l'invention concerne une composition telle que définie ci-dessus à titre de médicament, de vaccin, ou de réactif de diagnostic *in vitro* et/ou *in vivo,* pour l'induction ou le diagnostic, chez un mammifère, du passage d'un état immunitaire de type Th2 vers un état immunitaire de type Th1.

Un objet additionnel de l'invention concerne une composition telle que définie ci-dessus à titre de médicament, de vaccin, ou de réactif de diagnostic *in vitro* et/ou *in vivo,* pour l'induction ou le diagnostic, chez un mammifère, d'anticorps spécifiques et plus particulièrement d'isotypes IgG2.

Un problème additionnel que se propose de résoudre la présente invention est de disposer d'un vaccin capable de conférer une immunoprotection croisée vis-à-vis des différentes espèces de leishmaniose. L'importante communauté antigénique partagée par les leishmanies permet d'envisager le développement d'un vaccin unique polyvalent, constitué d'immunogènes communs et hautement conservées. Le fait de cibler comme vaccin un (des) antigène(s) commun(s) à toutes les espèces de leishmanies devrait sans aucun doute représenter un réel avantage en terme de vaccination croisée.

Ainsi, un quatrième objet de l'invention concerne un vaccin contre les leishmanies comprenant au moins:
- un épitope de séquence choisi parmi les séquences SEQ ID N°1 à 10; ou
- un composé peptidique comprenant 1, 2, 3 ou 4 des épitopes de séquences SEQ ID N°1 à 10, éventuellement séparés par un espaceur tel que défini ci-dessus; ou
- un composé peptidique de séquence choisie parmi SEQ ID N°11 à 13.

Ledit vaccin est avantageusement utilisé pour une vaccination prophylactique et thérapeutique dirigée contre une ou plusieurs des leishmanies choisies parmi Leishmania *donovani, Leishmania infantum, Leishmania chagasi, Leishmania mexicana, Leishmania amazonensis, Leishmania venezuelensis, Leishmania Tropica, Leishmania major, Leishmania* aethiopica, Leishmania *(Viannia) braziliensis, Leishmania (Viannia) guyanensis, Leishmania (Viannia) panamensis, Leishmania (Viannia) peruvian.*

Ledit vaccin objet de l'invention est avantageusement destiné à l'Homme, aux canidés, aux félidés et aux équidés. Préférentiellement, le vaccin objet de l'invention est destiné à l'Homme et aux chiens.

De préférence, le vaccin selon l'invention comprend au moins deux épitopes ou composés peptidiques dont au moins un épitope est choisi parmi:
- un épitope de séquence choisi parmi les séquences SEQ ID N°1 à 10; ou
- un composé peptidique comprenant 1, 2, 3 ou 4 des épitopes de séquence SEQ ID N°1 à 10, éventuellement séparés par un espaceur tel que défini ci-dessus; ou
- un composé peptidique de séquence choisie parmi SEQ ID N°11 à 13.

De préférence encore, le vaccin selon l'invention comprend au moins deux épitopes ou composés peptidiques choisis parmi:
- un épitope de séquence choisie parmi les séquences SEQ ID N°1 à 10; ou
- un composé peptidique comprenant 1, 2, 3 ou 4 des épitopes de séquences SEQ ID N°1 à 10, éventuellement séparés par un espaceur tel que défini ci-dessus; ou
- un composé peptidique de séquence choisie parmi SEQ ID N°11 à 13.

En outre, un problème additionnel que se propose de résoudre la présente invention est de disposer d'un vaccin capable d'assurer une couverture vaccinale à l'échelle mondiale. Ce candidat vaccinal doit donc préférentiellement être reconnu par l'ensemble des molécules du Complexe Majeur d'Histocompatibilité de classe I et de classe II majoritairement représentées dans les populations humaines les plus gravement touchées par ces affections.

Dans ce contexte, outre les aspects sécuritaires, d'efficacité et de faisabilité industrielle propres au développement d'une vaccination, un vaccin contre les leishmanioses devrait en outre satisfaire à un certain nombre d'exigences plus spécifiques. L'élaboration d'un tel vaccin s'accompagne de difficultés supplémentaires dues à la complexité du cycle de vie des leishmanies, à la diversité génétique (plus de 20 espèces de leishmanies sont responsables des infections humaines), aux mécanismes d'échappement extrêmement sophistiqués que ces pathogènes ont su élaborer au cours de l'évolution pour s'établir dans des cellules clés de l'immunité en déjouant et en utilisant à leurs profits les réponses immunitaires de l'hôte. A cela s'ajoute l'extrême diversité et pauvreté des populations touchées par ces maladies.

Une difficulté additionnelle pour atteindre le dernier objectif de la présente invention n'est pas seulement de conduire une étude sur la leishmaniose viscérale et d'obtenir des résultats transposables avec efficacité et innocuité au chien et à l'Homme, mais d'obtenir un vaccin unique adapté à la prévention et au traitement de toutes les espèces de Leishmania chez les mammifères, et prenant en compte la complexité du polymorphisme HLA de l'Homme.

Ainsi, selon un mode préféré de réalisation de l'invention, le vaccin selon l'invention comprend avantageusement, d'une part, au moins un composé peptidique choisi parmi SEQ ID N°9 et 10;
et d'autre part, au moins un composé peptidique choisi parmi les séquences SEQ ID N°11 à 13,
lesdites séquences étant telles que définies ci-dessus. Bien entendu, comme indiqué précédemment, selon l'invention, les composés peptidiques ci-dessus incluent leurs dérivés analogues, mutéines et homologues.

De préférence, parmi les dérivés envisageables, on choisira préférentiellement les dérivés peptidiques des composés ci-dessus comportant au moins cinq acides aminés contigus.

L'approche multiépitopique d'un vaccin peptidique conduit à la mise au point d'un vaccin destiné à l'ensemble de la population mondiale. Pour assurer une bonne couverture de la population mondiale et au regard de la grande variabilité du phénotype HLA (complexe majeur d'histocompatibilité humain) entre les individus, les fragments antigéniques immunogènes (peptides) de longueur suffisante doivent de préférence contenir une série d'épitopes aptes à être présentés par plusieurs types de molécules HLA-I et -II.

L'identification d'épitopes, au sein des antigènes majeurs et conservés de Leishmania, reconnu par les cellules T ouvrent de nouvelles perspectives en matière d'immunothérapie et d'immunoprophylaxie des Leishmanioses.

Ainsi la détection de premiers peptides portés par la partie carboxy-terminale de l'antigène majeur PSA a permis d'obtenir chez les chiens un candidat vaccinal peptidique qui induit une réponse à médiation cellulaire conférant un bon niveau de protection à l'encontre d'une infection expérimentale à Leishmania infantum (brevet FR2829767) ainsi qu'une réponse anticorps spécifique IgG2 importante permettant de distinguer en zone d'endémie un hôte vacciné d'un hôte non vacciné mais infecté (brevet FR2932802) .

Comme cela est montré aux exemples 3 et 4, le Demandeur a pu mettre en évidence que les épitopes ou composés peptidiques SEQ ID N°1 à 8, SEQ ID N°11 à 13 sont des composés peptidiques présentant une affinité pour les molécules du Complexe Majeur d'Histocompatibilité de classe I.

De même, le Demandeur a mis en évidence que les épitopes ou composés peptidiques SEQ ID N°9 et 10 sont reconnus par les molécules du Complexe Majeur d'Histocompatibilité de classe II.

De façon surprenante, le Demandeur a mis en évidence que l'ensemble de ces peptides est commun à toutes les espèces de *Leishmania.*

De préférence, le vaccin selon l'invention comprend donc d'une part, au moins un ou deux composés peptidiques choisis parmi SEQ ID N°9 et 10;
et d'autre part, au moins un, deux ou trois composés peptidiques choisis parmi les séquences SEQ ID N°11 à 13, lesdites séquences étant telles que définies ci-dessus.

De préférence encore, le vaccin selon l'invention comprend, en combinaison, les cinq composés peptidiques multiépitopiques SEQ ID N°9 à 13, lesdites séquences étant telles que définies ci-dessus. Bien entendu, comme indiqué précédemment, selon l'invention, les composés peptidiques ci-dessus incluent leurs dérivés analogues, mutéines et homologues.

Ces cinq composés peptidiques prennent en compte l'extrême diversité des espèces de leishmanies et le niveau élevé du polymorphisme HLA.

Les composés peptidiques qui ont été avantageusement sélectionnés sont destinés à induire la prévention ou le traitement des leishmanioses chez les mammifères, notamment chez l'Homme, dont l'immunité protectrice dépend de la stimulation des lymphocytes de type Th1 (cellules T auxiliaires CD4+) et des lymphocytes T cytotoxiques CD8+. Cet état immunitaire Th1 et cytotoxique, est en pleine corrélation avec un état de résistance aux pathogènes intracellulaires tels *Leishmania, Trypanosoma, Candida, Mycobacterium* et *Listeria.*

Pour l'élaboration spécifique de ces composés peptidiques, le Demandeur a, par de nombreuses expériences, dû considérer, rechercher et caractériser les polymorphismes HLA les plus fréquemment rencontrés chez les populations humaines exposées aux infections leishmaniennes, auxquels sont contraintes les réponses à médiation cellulaires de l'hôte (HLA-I : réponse cytotoxique CD8+ ; HLA-II : réponses auxiliaires CD4+).

Le polymorphisme HLA représentatif de la population humaine mondiale a été déterminé et les supertypes pour la conception d'un vaccin leishmanioses ont été sélectionnés.

L'alignement des séquences amino- et carboxy-terminales des PSA (pour Promastigote Surface Antigen) des principales espèces de leishmanies capables d'infecter, couplé à des méthodes de prédiction *in silico* reposant sur les propriétés de liaison de séquences peptidiques vis-à-vis des molécules HLA, a conduit à :
- localiser les régions les plus conservées entre les PSA (parties carboxy- et amino- terminale) des différentes espèces de leishmanies ;
- établir les cartes de prédiction *in silico* des zones riches en épitopes à forte et moyenne affinités pour les molécules HLA classe I et HLA classe II sur les séquences de PSA (parties carboxy- et amino- terminale) ;
- sélectionner des séquences peptidiques consensus riches en épitopes HLA-I et HLA-II et à concevoir des séquences multi-épitopiques HLA classe I consensus et optimisées, présentant un bon processus de présentation des épitopes et une conservation des scores de liaison pour construire des composés peptidiques multiépitopiques (de 18 à 28 acides aminés) dans l'objectif de multiplier les chances d'activation du système immunitaire.

Comme cela est démontré aux exemples 1 à 6 ci-dessous, la combinaison spécifique des cinq composés peptidiques SEQ ID N° 9 à 13 permet de répondre avec une grande efficacité aux inconvénients et insuffisances des modèles proposés antérieurement. Comme cela est montré aux exemples 7 et 8 le Demandeur a évalué l'efficacité de ce vaccin peptidique *in vivo* et dans des essais cliniques chez le chien et dans des tests ex *vivo* sur des cellules humaines.

De façon avantageuse, le vaccin objet de l'invention comprend en outre un adjuvant permettant de préférence d'accroitre la réponse immunitaire aux composés peptidiques objet de l'invention. Les adjuvants sont, le plus souvent, des substances minérales, huileuses ou dérivées de certains microorganismes. De préférence, le ou les adjuvants associés aux composés peptidiques objet de l'invention induisent une réponse et médiation cellulaire et sont choisis parmi les classes TLR3, TLR4, TLR5, TLR7, TLR8, TLR9, les saponines, les émulsions huile dans eau ou eau dans huile, les polysaccharides, les liposomes cationiques, les virosomes ou les poly-electrolytes. Parmi ces différentes classes, on peut citer, à titre d'exemples non limitatifs les composés Poly I:C, Poly A:U, MPL, RC530, GLA, flagelline, Imiquimod, Resiquimod, CpG, IC41, QS21, squalane, squalène, tocopherol, inuline, DDA et polyoxidonium, QA21, la saponine, la quil-A, ou tout autre dérivé de ceux-ci connu de l'homme du métier, pris seuls ou en mélange.

De façon préférentielle, le rapport composés peptidiques/adjuvant est compris entre 3/1 et 3/3.

Selon un mode particulier de réalisation de l'invention, le vaccin comprend en outre un composé peptidique cyclisé par pont disulfure composé d'une séquence de 34 acides aminés et désigné E34P.

Ce composé peptidique de 34 acides aminés (E34P) est composé de la séquence SEQ ID N° 14 suivante: E-D-EH-K-G-K-Y-C-R-L-G-N-D-C-R-T-T-E-P-T-T-T-A-T-P-R-G-T-P-T-P-A-P.

Ce composé peptidique est cyclisé par pont disulfure entre la cystéine en position 9 (Cys 9) et la cystéine en position 15 (Cys 15). Le composé peptidique E34P est avantageusement capable d'induire une réponse anticorps importante permettant de discriminer en zone d'endémie un hôte vacciné d'un hôte non vacciné mais infecté. Cette cyclisation du composé peptidique est essentielle pour l'apparition des anticorps spécifiques IgG2, la cyclisation en pont disulfure entre les cystéines 9 et 15 induisant une conformation spécifique responsable de cette synthèse IgG2. En effet, la non cyclisation du composé peptidique en Cys 9 / Cys 15 n'induit qu'une synthèse peu importante en IgG2 parfois difficile à déceler.

Bien entendu, comme indiqué précédemment, selon l'invention, le composé peptidique E34P inclue ses dérivés analogues, mutéines et homologues tels que définis ci-dessus.

De façon particulièrement avantageuse, le vaccin selon l'invention comprend les composés peptidiques objets de l'invention dans le rapport suivant :
SEQ ID N°9 / SEQ ID N°10 / SEQ ID N°11 / SEQ ID N°12 / SEQ ID N°13 : 20 / 20 / 20 / 15 / 25.

De façon plus avantageuse encore, le vaccin selon l'invention comprend les composés peptidiques objets de l'invention ainsi que le composé peptidique E34P dans le rapport suivant :
SEQ ID N°9 / SEQ ID N°10 / SEQ ID N°11 / SEQ ID N°12 / SEQ ID N°13 / SEQ ID N°14: 20 / 20 / 20 /15 / 25 / 10.

Enfin, un dernier problème que se propose de résoudre la présente invention est de disposer d'un vaccin ayant la capacité d'induire des réponses immunitaires permettant de distinguer un hôte vacciné d'un hôte infecté. En effet, comme pour de nombreuses maladies parasitaires, le diagnostic clinique reste aléatoire car les symptômes sont peu spécifiques, souvent absents (il existe de nombreux porteurs asymptomatiques) et n'apparaissent vraiment que lors d'une phase très avancée de l'infection, qui devient alors extrêmement difficile à traiter. Le diagnostic sérologique qui consiste à mettre en évidence des anticorps spécifiques circulants est pratiqué en routine (technique d'immunofluorescence indirecte). Un vaccin lorsqu'il est administré, génère en général des anticorps spécifiques qui très souvent peuvent être aussi produits lors d'une infection. La présence de tels anticorps chez un hôte peut alors être confondante dans le sens où infection et vaccination possèdent la même signature.

Ainsi, selon un mode préféré de réalisation de l'invention, le vaccin comprend en outre au moins une signature. La signature peut être tout antigène capable de générer des anticorps spécifiques lors d'une immunisation mais non produits lors d'une infection.

A titre d'exemple non limitatif de signature utilisables selon l'invention, on peut citer le composé peptidique cyclisé par pont disulfure E34P décrit ci-dessus. Ce composé peptidique de 34 acides aminés (E34P) est composé de la séquence SEQ ID N° 14 suivante: E-D-EH-K-G-K-Y-C-R-L-G-N-D-C-R-T-T-E-P-T-T-T-A-T-P-R-G-T-P-T-P-A-P. Ce composé peptidique est cyclisé par pont disulfure entre la cystéine en position 9 (Cys 9) et la cystéine en position 15 (Cys 15).

Selon un mode de réalisation additionnel, l'invention concerne également une séquence nucléotidique codant pour:
- un épitope de séquence choisi parmi les séquence SEQ ID N°1 à 10; ou
- un composé peptidique comprenant 1, 2, 3 ou 4 des épitopes de séquence SEQ ID N°1 à 10, éventuellement séparés par un espaceur tel que défini ci-dessus; ou
- un composé peptidique de séquence choisie parmi SEQ ID N°11 à 13.

L'invention concerne également un vecteur d'expression comprenant au moins une séquence nucléotidique telle que définie ci-dessus, ainsi que les moyens nécessaires à leur expression.

Enfin, l'invention concerne un réactif de diagnostic comprenant:
- un épitope de séquence choisi parmi les séquence SEQ ID N°1 à 10; ou
- un composé peptidique comprenant 1, 2, 3 ou 4 des épitopes de séquence SEQ ID N°1 à 10, éventuellement séparés par un espaceur tel que défini ci-dessus; ou
- un composé peptidique
de séquence choisie parmi SEQ ID N°11 à 13.

La présente invention va maintenant être illustrée au moyen des exemples suivants :

### Exemple 1 : Analyse des supertypes HLA pour une efficacité à l'échelle mondiale

Afin de mettre au point des épitopes ou des composés peptidiques multi-épitopiques, le Demandeur a recherché, recensé, et caractérisé les supertypes HLA les plus fréquemment rencontrés dans le monde, notamment chez les populations humaines exposées aux infections leishmaniennes. Les tableaux 1 et Ibis ci-dessous représentent la fréquence moyenne des supertypes HLA-I et -II dans les pays les plus concernés par la leishmaniose, dans la population mondiale ainsi que les haplotypes associés.

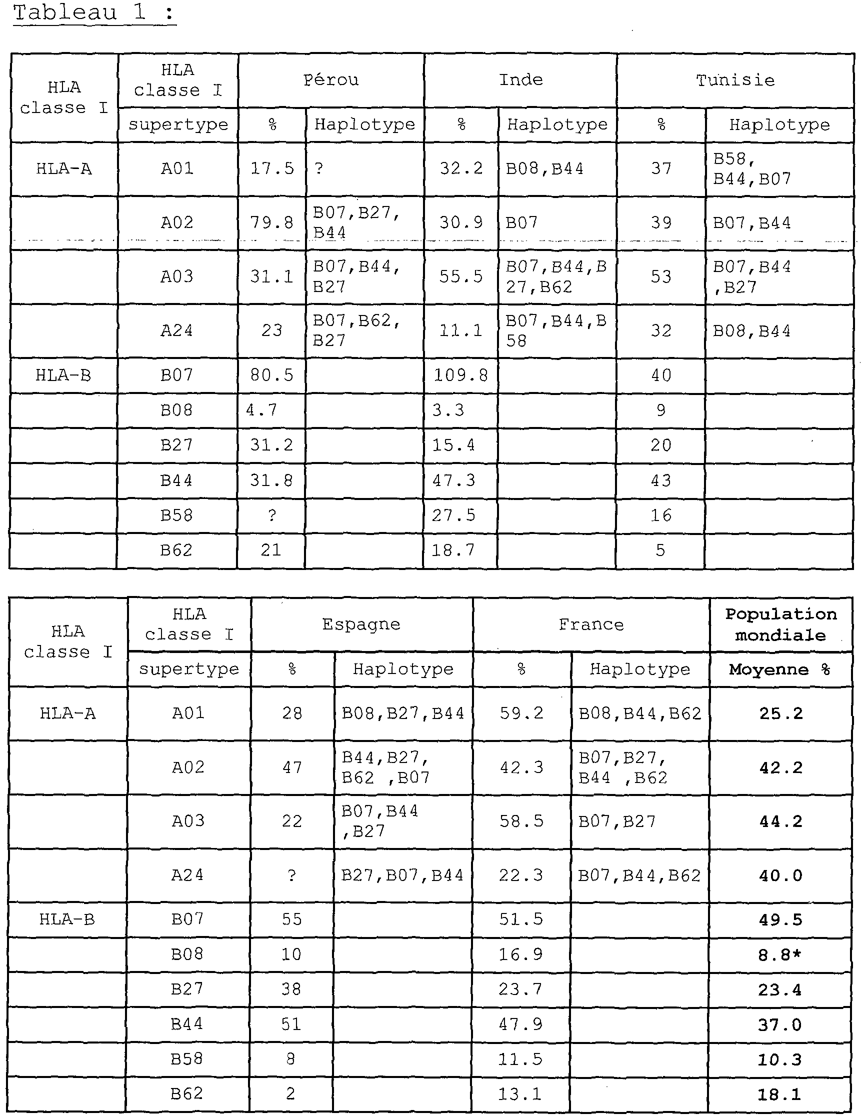

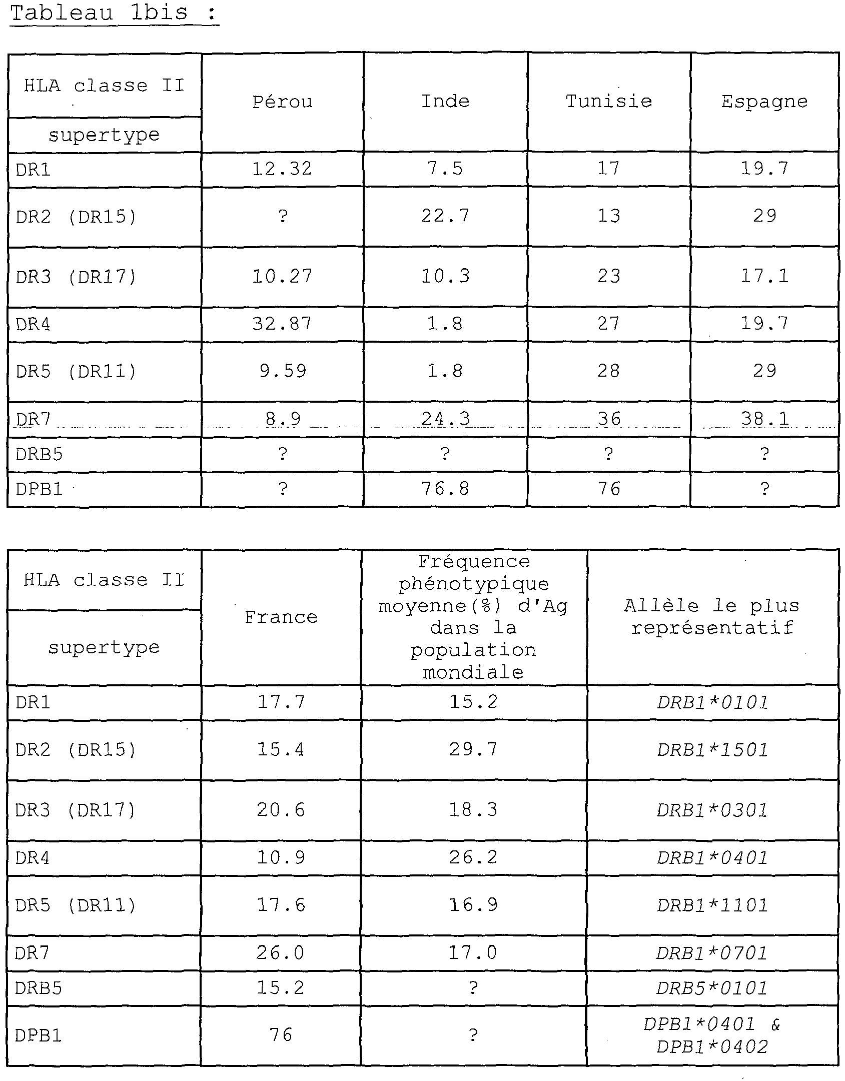

Les pourcentages calculés dans les tableaux 1 et Ibis ci-dessus sont évalués d'après Sette A. and Sidney J. Immunogenetics, 1999

Le Demandeur a ensuite déterminé les combinaisons de supertypes HLA de classe I à considérer pour la recherche et l'identification de composés peptidiques multi-épitopiques T capables d'assurer la couverture vaccinale des populations les plus gravement touchées par les leishmanioses.

Le tableau 2 ci-dessous met en évidence les supertypes sélectionnés et montre que la prise en compte, en combinaison, de plusieurs supertypes HLA, notamment pour HLA-I, permet d'assurer une meilleure couverture vaccinale de la population mondiale.

**Tableau 2 :**

| Combinaisons de supertypes HLA | Couverture de la population estimée (Caucasiens + Noirs N.A + Japonais + Chinois + Hispaniques) |
|---|---|
| HLA-A02, A03 and B7 | 86.2% |
| HLA-A02, A03, B07, A24, B44 and A01 | 99.2% |
| HLA-A02, A03, B07, A24, B44, A01, B27, B62 and B58 | 99.8% |

Comme pour le tableau 1, les pourcentages calculés dans le tableau 2 ci-dessus sont évalués d'après Sette A. and Sidney J. Immunogenetics, 1999]

Comme cela est montré au tableau 2 ci-dessus, les supertypes HLA-I qui doivent être reconnus par les composés peptidiques entrant dans la composition d'un candidat vaccin selon l'invention pour assurer une couverture vaccinale acceptable contre les leishmanioses sont donc les supertypes HLA-A02, A03 et HLA-B07. De préférence, pour assurer une bonne couverture vaccinale, les supertypes HLA-I qui doivent être reconnus par les composés peptidiques sont les supertypes HLA-A01, A02, A03, A24 et HLA-B07 et B44, de préférence encore les supertypes HLA-A01, A02, A03, A24 et HLA- B07, B27, B44, B58 et B62.

De façon particulièrement avantageuse, les supertypes HLA-I qui doivent être reconnus par les composés peptidiques sont les supertypes HLA-A01, A02, A03, A24 et HLA-B07, B08, B27, B44, B58 et B62 listés dans le tableau 1 ci-dessus.

En ce qui concerne les supertypes HLA-II, les supertypes sélectionnés sont les supertypes HLA-DR1, DR2, DR3, DR4, DR5, DR7, DRB5 et DPB1.

Compte tenu de ce qui précède, le Demandeur a pu établir qu'un vaccin comprenant des épitopes ou des composés multi-épitopiques reconnus par l'ensemble des supertypes HLA ci-dessus assurerait donc une couverture vaccinale optimale à l'échelle mondiale.

### Exemple 2 : Identification des séquences consensus dans les différentes PSA (pour Promastigote Surface Antigen).

Le Demandeur a répertorié puis étudié les séquences amino- et carboxy- terminales des PSA des principales espèces de leishmanies capables d'infecter l'Homme, afin de mettre en évidence des zones très conservées, également appelées zones « consensus ».

Les séquences amino-terminales des PSA (Nter-PSA) des principales espèces de leishmanies sont répertoriées ci-dessous. Elles sont représentées sans le peptide signal des voies d'excrétion-sécrétion.

Nter Lma A3B [protein=LaPSA-38S;acc=ACY70940]
AGTSDFTEAQQTNTLTVLQAFARAIPALGDTWTGSDFCSWKHIICDSPGVGV
WMGDVDYTGTLPEMPASVDYKDV

Nter Li IJ11 [protein=LiPSA-50S; acc=ACY70941]
AVKDDFTAAQRTNTLAVLEAFGRAIPELGKLWKGDDFCFWESVVCDVTEVYL
WEIGATYTGTLPEMPVDVDYTAV

Nter PSAb Ldd [protein=LdPSAb;acc=AAY96325]

AGKDDFTPEQRTNTLAVLQAFGRAIPELGKKWIGNDFCAWEYIKCYSSAVSL
WMDSVDYAGTLPEMPAGVDYKHV

Nter PSA2 LmjF12.0980
[protein=PSA2;acc=XP 001681679]
AGTGDFTAAQRTNTLAVLQAFGRAIPKLGEKWAGNDFCSWEAVLCNAPDVYV
SGISPTYAGTLPEMPVNVDYRHV

Nter PSA2  LmjF12.0755
[protein=PSA2;acc=XP 001681698]
GTNDFTAAQRTNTLAVLQAFGRAIPELGEKWAGNDFCSWEFIVCNVIGVNVR
GISPTYAGTLPEIPVNVDYRHV

Nter PSA2 LTropica [protein=PSA2;acc= AAF80491]
AGTGDFTDEQRTNTLAVLQAFGSAIPELGEKWTGDDFCSWDHVTCVSLSVFV
RLDVSTYAGTLPEMPVGVDYSHV

Nter GP46 LChagasi 627aa [protein=GP46;acc=AAR11511]
AGISDFTSAQQLNTLAVLQAFGRAIPELGKLWTGDDFCSWDFIKCVSSGARV
WLKGATYAGTLPQMPAGVDYKHV

Nter GP46 LChagasi 417aa [protein=GP46;acc=AAB62271]
VKEDNLTETQKSNTLAVLQAFGRAIPEVKKLWKGDDFCSWKYVVYCYPSAVGV
LMDSVDYAGSLPEMPAGVDYTNV

Nter SurfaceAntigenProt Lbraz912aa
[protein=surface antigen protein;acc=XP 001563125]
TVTGDFTAEQQSNTLAVLQAFAGAIPELQSTWSGSDFCSWGGVTCAVSSVRV
AGINAMYTGTLPEMPAGVDYANV

Nter lca08prot1 Lbraz 793aa (field strain) 750B FW
and REV
TVTGDFTAEQQSNTLAVLQAFAGAIPELQSTWSGSDFCSWGGVTCAVSSVRV
AGINAMYAGTLPEMPAGVDYANV

Nter Ldd K59 (Field strain) AP1-P3
AGKDDFTPEQRTNTLKVLQAFGRAIPELGKKWIGNDFCAWEYIKCYSSAVSL
WMDSVDYAGTLPGMPAGVDYKHV

Nter Ldd8 cl2 montp2 IRD [reference strain]P1-P3
AGKDDFTPEQRTNTLKVLQAFGRAIPELGKKWIGNDFCAWEYIKCYSSAVSL
WMDSVDYAGTLPEMPAGVDYKHV

Nter Ldi K263 cl2 Montp IRD [reference strain] P1-P3
AVKDDFTAAQRTNTLAVLEAFGRAIPELGKLWKGDDFCFWEPVVCDVTEVYL
WEIGATYTGTLPEMPVDVDYTAV

Les séquences carboxy-terminales des PSA (Cter-PSA) des principales espèces de leishmanies sont répertoriées ci-dessous. Elles sont représentées sans le peptide signal de l'ancrage-GPI. Le glycosylphosphatidylinositol ou GPI, permet l'ancrage au niveau extracellulaire de diverses molécules, en particulier des protéines (enzymes) aux membranes cellulaires. C'est une charnière qui permet à la molécule de rester fixée à la membrane et de jouer son rôle.

Cter A3B [protein=LaPSA-38S;acc=ACY70940]
KDRLDVTIEEWHMGEDCKLANACRPTAAPGTTTTNPPTTTGTPAASSTPSPG
SGCEVDGCEVCEGDSAARCARCREGYSLTDEKTCLANHDGGVAAAS

Cter IJ11 [protein=LiPSA-50S; acc=ACY70941]
KAGLVVEIEDKHTGNSCIAGADCATTTTTTTEPTSTASPTATPTSAPETECE
VDGCEVCDGDSAARCARCREGYFLTDERTCLVYRDGGVVAVSIG

Cter PSAb Ldd [protein=LdPSAb;acc=AAY96325]
TQRIAVTIEDEHKGRNCKLENKCRQAAPTTTATPTATPTLAPETECEVDGCE
VCEGDSAARCARCGAGYFLTSEKTCRANGDGGVAAVSS

Cter PSA2 LmjF12.0980
[protein=PSA2;acc=XP 001681679]

KAGLVVDIEDKHKGSDCLAAKDCTTTTTKPSATTATTPNLTNFPPTPRTTTE
PLTTTSTEAPAEPTTTTEAPAEPTTTATPTNTPTPAPETECEVDGCEVCEGD
SAARCARCREDYFLTDEKTCLKHND

Cter PSA2  LmjF12.0755
[protein=PSA2;acc= XP 001681698]
KAGLVVGIEDKHKGSDCLAAKDCTTTTTKPPTTTTTPTKPPATTTTEAPAEP
TTTTEAPAEPTTTTEAPAEPTTTTEAPAEPTTTTEAPAEPTTTATPTNTPTP
APETECEVDGCEVCEGDSAARCARCREDYFLTDERTCLVYCD

Cter PSA2 Ltropica [protein=PSA2;acc= AAF80491]

KANLVVDIEDEHKGTNCLAGPDCTTTTTTTTTTKPPTTTTTTTTKPPTTTTTTT

TKPPTTTTTTTTTKPPTTTTTTTTTEPPATTTTTTTKPPTTTTTTEAPAEPTTTA

TPTNTPTPAPETECEVDGCEVCEGDSAARCARCGDDYFLTDEKTCLVYCDGG

VAAASSGV

KAGLVVSIEDKHTGNNCIASGECTTTTTTTTTTTKPPTTTTTTKPPTTTTTTT
KPPTTTTTKPPTTTTTTTKPPTTTTTTKPPTTTTTKPPTTTTTTTKPPTTTTT
KPPTTTTTKPPTTTTTEAPSEPTTTASPTATPTPAPETECEVDGCEVCDGDSA
ARCARCREGYFLTDXRTCLVYCDGGVVAAT
Modif : TDERTCLVYCDGGVVAAT

Cter GP46 LChagasi 417aa [protein=GP46;acc=AAB62271]
KRKLMVTIEADHEGRNCKLENKCRPAAPTTTTTTTSTTTKRATASTSTTTTT
TVPPTLPSTTATPTATPTPAPETECEVDGCEVCEEDSAARCARCREGYFVTS
EKTCLMTTDGGLAAALS

Cter SurfaceAntigenProt Lbraz912aa
[protein=surface antigen protein;acc=XP 001563125]
KPGLVVSIEDEHMGNDCTTENICPTTTTTTTTTTTSTEPPAVSTTTTTŠTEP
PAVSTTTTTSTEPPAVSTTTTTSTESPAVSTTTTTSTEPPAVSTTTTTSTEP
PAVSTTTTTSTEPPAVSTTTTTTSTEPPAVSTTTTTTSTEPPAVSTTTTTST
ESPAVSTTTTTSTEPPAVSTTTTTSTEPPAVSTTTTLSPDTRCEVDGCAKCE

DGSSVRCARCHDDYYLMDDKTCLVYCADDVAAAPSGVLTAAVVCVVTLFSMG
LVM

Cter lca08prot1 Lbraz 793aa (field strain) 750B FW
and REV
KPGLVVSIEDEHMGNDCTTENICPTTTTTTTTTTTSTEPPAVSTTTTTTSTEP
PAVSTTTTTSTESPAVSTTTTTSTESPAVSTTTTTSTEPPAVSTTTMTSTES
PAVSTTTTLSPDTRCEVDGCAKCEDDSSVRCARCHDDYYLMDDKTCLVYCAD
DVAAAPSGVLTAAVVCVVTLFSMGLVMGCRGCPVQTT

Cter Ldd K59- (Field strain) AP1-P3
TQRIAVTIEDEHKGRNCKLENKCRQAAPTTTATPTATPTLAPETECEVDGCE
VCEGDSAARCARCREGYFLTSEKTCRANGDGGVAAVSS

Cter Ldd8 cl2 montp2 IRD [reference strain]P1-P3
TQRIAVTIEDEHKGRNCKLENKCRQAAPTTTATPTATPTLAPETECEVDGCE
VCEGDSAARCARCREGYFLTDEKTCL

Cter Ldi K263 cl2 Montp IRD [reference strain] P1-P3
KAGLAVEIEDKHTGNSCIAGADCATTTTTTTEPTSTASPTATPTSAPETECE
VDGCEVCDGDSAARCARCREGYFLTDEKTCL

### Identification de séquences consensus amino-terminales des PSA des différentes espèces de leishmanies :

Les séquences amino-terminales des PSA (Nter PSA) ont été alignées dans le but de localiser les régions les plus conservées entre les PSA des différentes espèces de leishmanies.

Le tableau 3 ci-dessous illustre l'alignement des séquences amino-terminales des différentes PSA (Nter-PSA). Les acides aminés représentés en blanc sur fond noir mettent en évidence les séquences présentant le plus d'homologie et qui sont les plus conservées. Il s'agit de séquences consensus.

Les différents symboles représentés sous les séquences mettent en évidence le degré d'homologie ou d'identité entre les différentes séquences alignées.

Le symbole « * » indique que l'acide aminé du rang considéré est le même pour chacune des séquences, on parle alors d'identité. Le symbole « : » indique une forte homologie et le symbole « . » indique, quant à lui, une faible homologie.

L'absence de symbole indique l'absence d'homologie. Le « N- » en amont des séquences alignées dans le tableau 3 indique qu'il s'agit de l'extrémité amino-terminale desdites séquences.

Les séquences étudiées proviennent de différents types de leishmanies. Les différents sigles du tableau 3 ci-dessous indiquent de quel type de leishmanie proviennent les séquences étudiées :« LMJ » pour Leishmania major, « LDI » pour L. infantum, « LDD » pour L. donovani, « LCHA » pour L. chagasi, « LAMA » pour L. amazonensis, « LBRA » pour L. braziliensis et « LTRO » pour L. tropica.

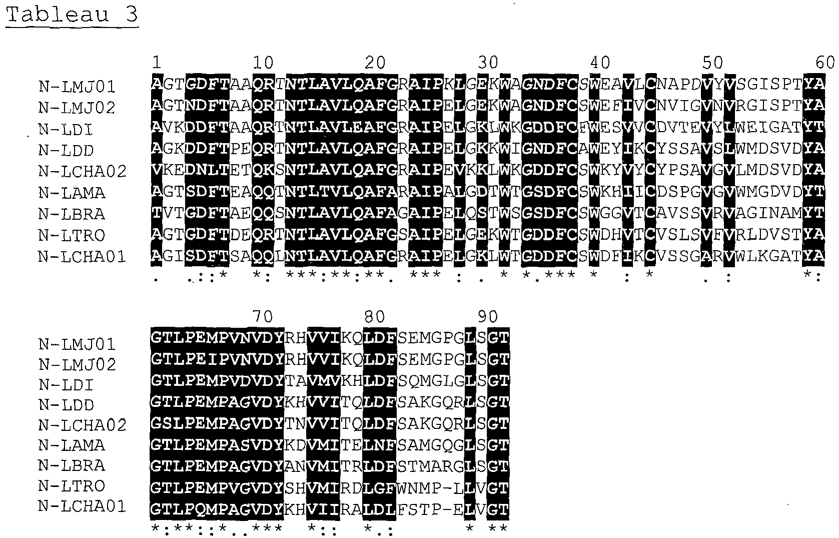

Suite à l'alignement de séquences ci-dessus, le Demandeur a pu établir les zones pour lesquelles les séquences présentent le plus d'homologie.

### Identification de séquences consensus carboxy-terminales des PSA des différentes espèces de leishmanies :

De la même façon, les séquences carboxy-terminales des PSA (Cter-PSA) ont été alignées. Le tableau 4 ci-dessous illustre l'alignement de la partie carboxy-terminale des séquences des différentes PSA.

Comme pour le tableau 3, les acides aminés représentés en blanc sur fond noir mettent en évidence les séquences présentant le plus d'homologie et qui sont les plus conservées, ou séquences consensus.

Le « C- » en amont des séquences alignées dans le tableau 4 indique qu'il s'agit de l'extrémité carboxy-terminale desdites séquences.

Les différents types de leishmanies étudiés sont les mêmes que ceux étudiés dans le tableau 3. Les sigles du tableau 4 permettent de les identifier.

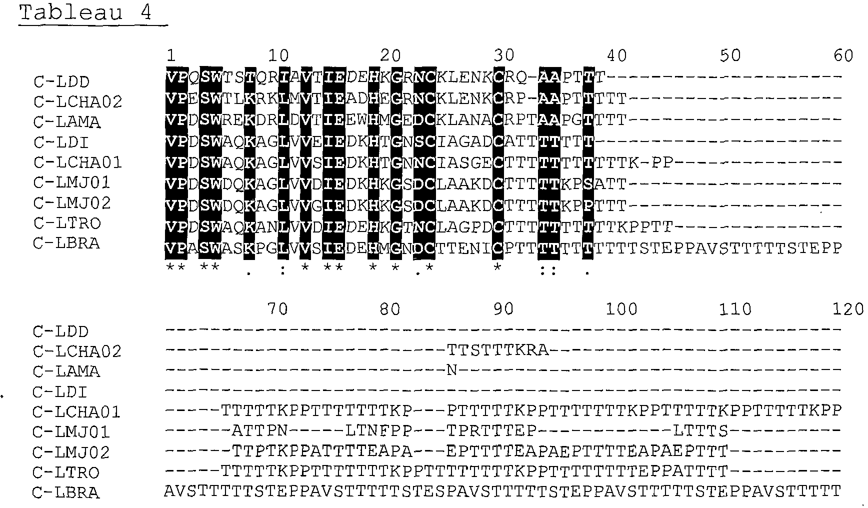

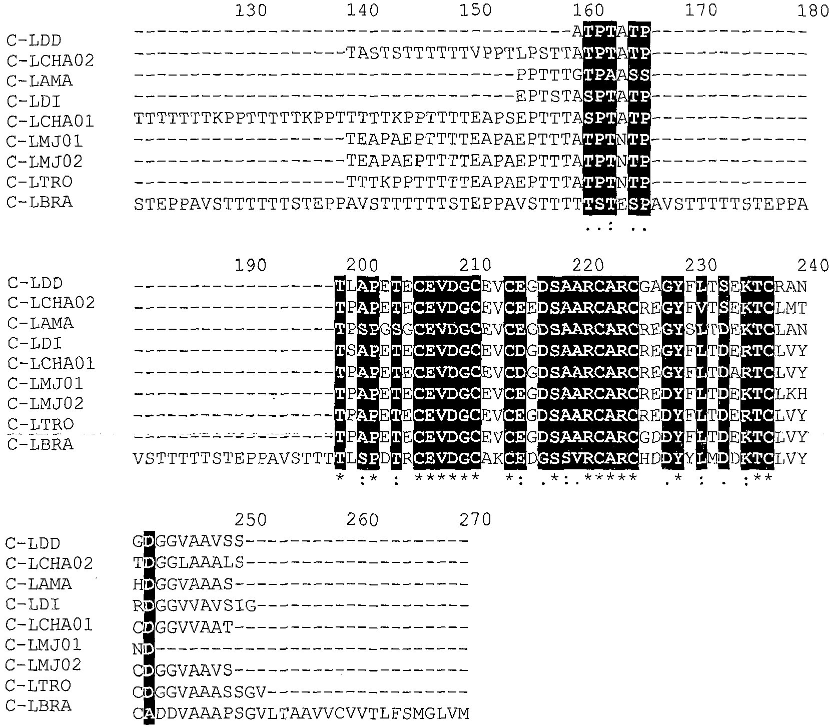

Suite à l'alignement de séquences ci-dessus, le Demandeur a pu établir deux zones principales pour lesquelles les séquences présentent le plus d'homologie.

Les alignements ci-dessus ont permis de localiser des zones « consensus », c'est-à-dire les zones les plus conservées entre les PSA des différentes espèces de leishmanies.

### Exemple 3 : Sélection et optimisation des épitopes en fonction de leur affinité avec les différentes molécules HLA classe I et HLA classe II.

Les différents dérivés de ces séquences « consensus » ont été testés grâce à des méthodes de prédiction *in silico* reposant sur les propriétés de liaison de séquences peptidiques vis-à-vis des molécules HLA. Des cartes de prédiction *in silico* des zones riches en épitopes à forte et moyenne affinités pour les molécules HLA classe I et HLA classe II sur les séquences des PSA (parties carboxy- et amino- terminale) ont été réalisées.

Les figures, 1, 2 et 3 illustrent la localisation des épitopes HLA sur les séquences de différentes PSA.

Ces résultats ont permis la sélection et l'optimisation de séquences peptidiques consensus riches en épitopes HLA-I et HLA-II.

A l'issu de ces tests, les 10 épitopes suivants ont été retenus :
SEQ ID N°1 : T-P-E-Q-R-T-N-T-L (épitope HLA-B07) ;
SEQ ID N°2 : E-L-G-K-K-W-I-G (épitope HLA-B08) ;
SEQ ID N°3 : T-L-P-E-M-P-V-G-V (épitope HLA-A02) ;
SEQ ID N°4 : P-E-M-P-A-G-V-D-Y (épitope HLA-A01) ;
SEQ ID N°5 : A-R-G-R-E-G-Y-F-L (épitope HLA-B27) ;
SEQ ID N°6 : A-R-G-A-R-G-R-E-G-Y (épitope HLA-B44) ;
SEQ ID N°7 : A-R-G-A-R-G-R-E-G (épitope HLA-B27) ;
SEQ ID N°8 : E-G-Y-F-V-T-D-E-K (épitope HLA-A03) ;
SEQ ID N°9 : T-N-T-L-A-V-L-Q-A-F-G-R-A-I-P-E-L-G-K-K-W ; et

SEQ ID N°10 : E-G-Y-F-V-T-D-E-K-T-G-L-V-Y-R-D-G-G-V-A-A-
A-S-S-G .

### Exemple 4 : Construction de composés peptidiques multi-épitopiques.

### a. Composés peptidiques multi-épitopes HLA-I :

Les épitopes présentant l'affinité la plus élevée pour les molécules HLA-I ont été assemblés en composés peptidiques multi-épitopiques (de 18 à 28 acides aminés) dans l'objectif de multiplier les chances d'activation du système immunitaire.

L'affinité des composés peptidiques SEQ ID N° 11 à 13 suivants :
SEQ ID N°11 : T-P-E-Q-R-T-N-T-L-T-V-E-L-G-K-K-W-I-G
SEQ ID N°12 : T-L-P-E-M-P-V-G-V-P-E-M-P-A-G-V-D-Y ; 
SEQ ID N°13 : A-R-G-R-E-G-Y-F-L-A-R-G-A-R-G-R-E-G-Y-E-G-
Y-F-V-T-D-E-K ;
 pour les supertypes HLA-I A01, A02, A03 (A11), A24, B07, B08, B27, B44 (B18), B58 et B62 a ensuite été déterminée par le Demandeur. Les résultats sont regroupés dans le tableau 5 ci-dessous.

**Tableau 5 : Composition en acides aminés et en épitopes des trois composés peptidiques HLA-I sélectionnés**

| Localisation sur PSA | **Peptide classe I (A, B ou C)** | **HLA classe I supertype** | Epitope inclus dans peptides sélectionnés | Séquences Epitopes | Score de liaison |
|---|---|---|---|---|---|
| Nter-zone 2 | **12** | **A01** | oui | **PEMPAGVDY** | 0,54 |
| Nter-zone2 | **12** | **A02** | oui | **TLPEMPVGV** | 0.97 |
| Cter-zone 2 | **13** | **A03 (A11)** | oui | **EGYFVTDEK** | 1,22 |
| | | A24 | | | |
| Nter-zone 1 | **11** | **B07** | oui | **TPEQRTNTL** | 1,57 |
| Nter-zone 1 | **11** | **B08** | oui | **ELGKKWIG** | 0,95 |
| Cter-zone 2 | **13** | **B27** | oui | **ARGARGREGY et ARGARGREG et ARGREGYFL** | 0.83 |
| | | | | | 0.93 |
| | | | | | 0.85 |
| Cter-zone 2 | **13** | **B44 (B18)** | oui | **ARGARGREGY et ARGARGREG** (clivage possible après G) | 0,72 |
| | | B58 | | | |
| | | B62 | | | |

A l'issu de cette étude, les 3 composés peptidiques multi-épitopes HLA-I de séquence SEQ ID N° 11 à 13 ont été sélectionnés.

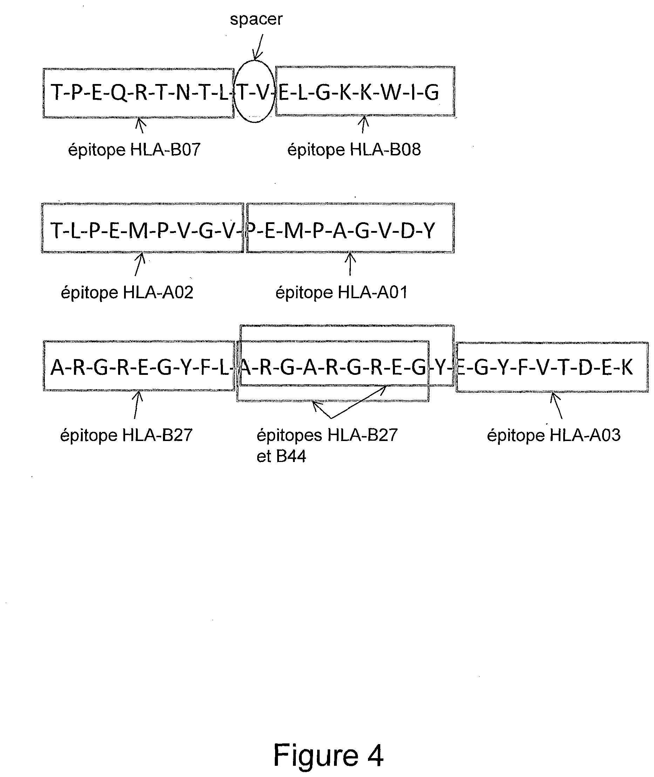

La figure 4 illustre la composition en épitopes des trois composés peptidiques HLA-I sélectionnés (affinité avec les supertypes HLA-I impliqués sur les 10 plus importants).

De façon avantageuse, ces composés peptidiques seront synthétisés sous leur forme palmytoyle (K).

### b. Composés peptidiques multi-épitopes HLA-II :

En outre, le Demandeur a réalisé une étude ayant pour but d'évaluer, pour les séquences consensus suivantes :
- Nter PSA : T-N-T-L-A-V-L-Q-A-F-G-R-A-I-P-E-L-G-K-K-W (SEQ ID N° 9) ;
- Cter-PSA-zone 1 : P-D-S-W-A-Q-K-A-G-L-V-V-T-I-E-DE ; et
- Cter-PSA-zone 2 : E-G-Y-F-V-T-D-E-K-T-G-L-V-Y-R-D-G-G-V-A-A-A-S-S-G (SEQ ID N° 10),
le nombre d'épitopes ayant une affinité haute/modérée avec les différents types HLA-II.

Les résultats de cette étude sont repris dans le tableau 6 ci-dessous.

Pour cette étude, le serveur NetMHCII 2.2 peut par exemple être utilisé pour prédire l'affinité de différents peptides avec les différents types HLA. Il prédit la liaison de peptides aux molécules HLA-DR, HLA-DQ, HLA-DP ainsi qu'aux allèles du CMH classe 2 de la souris en utilisant un réseau artificiel de neurones. Les pronostiques peuvent être obtenus pour 14 allèles HLA-DR couvrant les 9 supertypes HLA-DR, les 6 supertypes HLA-DQ, les 6 supertypes HLA-DP ainsi que les deux allèles H2 classe 2 de la souris.

Les valeurs de prédictions sont données en valeurs d'IC50 en nM et comme un classement en pourcentage dans un ensemble de 1 000 000 de peptides naturels aléatoires. Les peptides de liaisons forts et faibles sont indiqués dans les résultats.

**Tableau 6 :**

| **PSA** | **DR1** | **DR2 (DR15)** | **DR3** | **DR4** | **DR5 (DR11)** | **DR6 (DR13)** | **DR7** | **DRB5** | **CPB1** |
|---|---|---|---|---|---|---|---|---|---|
| **Nter** | 1 haute / 1 modérée | 0/2 | 0/0 | 0/1 | 0/0 | 1/0 | 0/1 | 1/2 | 0/1 |
| **Cter-zone 1** | 0/1 | 0/0 | 0/0 | 0/0 | 0/0 | 0/0 | 0/0 | 0/0 | 0/0 |
| **Cter-zone 2** | 0/2 | 0/1 | 0/2 | 0/2 | 0/1 | 0/0 | 0/1 | 0/0 | 0/0 |

Suite à ces résultats, les deux composés peptidiques multi-épitopes HLA-II de séquences SEQ ID N°9 et 10 ont été sélectionnés.

Les tableaux 7 et 8 ci-dessous représentent la concentration en peptide compétiteur (nM) (SEQ ID N°9 ou SEQ ID N°10) pour laquelle 50% de la liaison du peptide de référence aux molécules HLA-DRB1 et aux molécules HLADRB3, -DRB4, -DRB5 et HLA-DP4, est inhibée (IC50).

Pour chacun des tableaux 7 et 8, les résultats sont issus de deux expériences indépendantes.

**Tableau 7 :**

| | DR1 | DR3 | DR4 | DR7 | DR11 | DR13 | DR15 |
|---|---|---|---|---|---|---|---|
| Référence | 2,1 | 150 | 9 | 5 | 62 | 100 | 65 |
| | 2.5 | 190 | 12 | 6,5 | 50 | 50 | 75 |
| SEQ ID N°9 | 1,8 | >10 000 | 25 | 10 | 120 | 5000 | 450 |
| | 1,7 | >10 000 | 22 | 30 | 200 | 1700 | 300 |
| SEQ ID N°10 | 550 | 35 | 18 | 8000 | 120 | 1800 | 180 |
| | 700 | 35 | 15 | 3500 | 200 | 900 | 60 |

Les valeurs indiquées correspondent aux IC50 obtenus pour chaque peptide étudié.

Les résultats sont reproductibles puisque les valeurs d'IC50 obtenues pour chaque expérience n'excèdent pas un facteur de trois.

Plus la valeur de l'IC50 est faible, meilleure est l'affinité du peptide pour la molécule HLA étudiée. Ainsi, il est important de noter que les peptides de référence n'ont pas tous la même capacité de liaison selon la molécule HLA considérée. Ces valeurs correspondent aux valeurs standards attendues.

Comme pour les résultats présentés ci-dessus, le tableau 8 illustre l'ensemble des valeurs d'IC50 obtenues pour les deux peptides testés et leur peptide de référence comme contrôle interne, pour la liaison aux molécules HLA-DRB3, -DRB4, -DRB5 et aux molécules HLA-DP401 et -DP402.

**Tableau 8 =:**

| | DRB3 | DRB4 | DRB5 | DP401 | DP402 |
|---|---|---|---|---|---|
| Référence | 35 | 11 | 9.5 | 8 | 4.5 |
| | 38 | 10 | 4 | 9 | 18 |
| SEQ ID N°9 | >10 000 | 13000 | 8 | >10 000 | 10 000 |
| | >10 000 | 15000 | 2,5 | >100 000 | >100 000 |
| SEQ ID N°10 | 150 | >10 000 | 10000 | >10 000 | 9000 |
| | 180 | >10 000 | 1500 | 50000 | >100 000 |

Le tableau 9 ci-dessous illustre l'activité relative des deux composés peptidiques SEQ ID N°9 et 10 pour les molécules HLA-DR, HLA-DRB et HLA-DP4 étudiées. L'activité relative est calculée à partir des deux tableaux 7 et 8 ci-dessus de la façon suivante : Ratio = (moyenne des valeurs d'IC50 obtenues pour le peptide testé)/(moyenne des valeurs d'IC50 obtenues avec le peptide de référence).

**Tableau 9 :**

| **Peptide** | **DR1** | **DR3** | **DR4** | **DR7** | **DR11** | **DR13** | **DR15** | **DRB3** | **DRB4** | **DRB5** | **DP401** | **DP402** |
|---|---|---|---|---|---|---|---|---|---|---|---|---|
| SEQ ID N° 9 | 1 | >187 | 2 | 3 | 3 | 41 | 5 | >274 | 1331 | 0,9 | >3727 | >2222 |
| SEQ ID N° 10 | 271 | 0,2 | 2 | 928 | 3 | 18 | 1 | 5 | >9535 | 688 | 5556 | 2000 |

Dans ce tableau 9, l'activité relative est définie par le ratio obtenu entre la moyenne des IC50 calculée avec le peptide compétiteur et la moyenne des IC50 calculée avec le peptide de référence. L'activité relative permet ainsi de comparer les propriétés de liaison du peptide testé à celle du peptide de référence qui correspond au peptide qui lie de façon la plus efficace une molécule HLA donnée. Ainsi, une activité relative de 2 signifie que le peptide testé lie 2 fois moins efficacement la molécule HLA étudiée que le peptide de référence. Ainsi, plus le ratio est faible, meilleure est l'affinité du peptide pour la molécule HLA considérée.

Par conséquent, selon le ratio obtenu, les composés peptidiques sont classés selon trois catégories :
- les composés peptidiques de haute affinité dont le ratio n'excède pas 20 ;
- les composés peptidiques de moyenne affinité, compris entre 21 et 101 ; et
- les composés peptidiques non affins avec un ratio supérieur à 102.

Au vu de l'ensemble des résultats obtenus, les deux composés peptidiques consensus présentent une bonne affinité pour les molécules HLA-DR4, -DR11, -DR13 et DR15.

Plus particulièrement, le composé peptidique SEQ ID N°9 se distingue du composé peptidique SEQ ID N°10 de par son affinité de liaison élevée aux molécules HLA-DR1, - DR7 et DRB5.

Au contraire, le composé peptidique SEQ ID N°10 présente une bonne affinité de liaison aux molécules HLA-DR3 et HLADRB3, alors que le composé peptidique SEQ ID N°9 n'est pas actif sur ces deux molécules HLA.

De façon plus générale, ces deux composés peptidiques sont des épitopes T potentiels capables d'activer des lymphocytes T CD4+ car ils se lient, à eux deux, à l'ensemble des molécules HLADRB1 majoritairement représentées dans la population mondiale, ainsi que deux molécules HLA-DR secondaires sur trois (HLA-DRB3 et - DRB5) .

Les cinq composés peptidiques retenus ont été sélectionnés pour assurer la couverture vaccinale et thérapeutique des populations les plus gravement touchées par les principales espèces de leishmanioses les plus pathogènes chez l'Homme.

La terminaison N des composés peptidiques peut être modifiée par addition d'un repère acide gras à partir d'une protéine N-acylée. La forme la plus commune de cette modification est l'addition d'un groupe palmitoyle (K). Cela rend le composé peptidique plus stable et augmente sa capacité à être présenté aux cellules du système immunitaire.

### Exemple 5 : Prédiction des sites de clivage par le protéasome

Le Demandeur a également réalisé une étude de la conservation et de la création de sites de coupure par le protéasome, permettant de favoriser le processus de présentation des épitopes par les molécules HLA-I. Cette étude a permis de concevoir des séquences multi-épitopiques HLA classe I consensus et optimisées, présentant un bon processus de présentation des épitopes et une conservation des scores de liaison des peptides aux molécules HLA. Les épitopes les plus affins pour les molécules HLA-I ont été assemblés pour construire des peptides multi-épitopiques, présentant de 18 à 28 acides aminés, en considérant plus particulièrement la conservation et/ou l'addition d'un espaceur, comme par exemple l'espaceur T-V présent dans la séquence SEQ ID N°11, des sites de clivage par l'immunoprotéasome des HLA de classe I, favorisant leur présentation au système immunitaire.

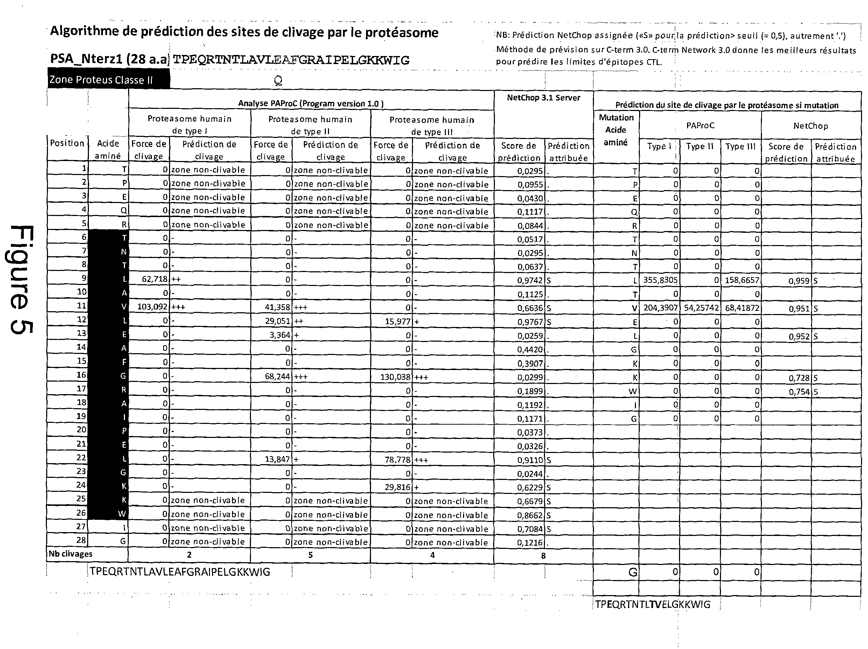

Les résultats de cette étude sont représentés dans la figure 5.

### Exemple 6 : Etude physico-chimique des composés peptidiques multi-épitopiques classe I et classe II issus des PSA de leishmanies et calcul des concentrations de ces composés.

Les composés peptidiques considérés lors de cette étude sont les composés peptidiques multiépitopiques HLA de classe I SEQ ID N°11 à SEQ ID N° 13 et les composés peptidiques multiépitopiques HLA classe II SEQ ID N°9 et 10.

Le composé peptidique épitope B « E-34-P » de SEQ ID N°14 est également étudié ici. Comme indiqué dans la description, il s'agit d'un composé peptidique de synthèse non natif, composé de la séquence de 34 acides aminés suivante :

E-D-E-H-K-G-K-Y-C-R-L-G-N-D-C-R-T-T-E-P-T-T-T-A-T-P-R-G-
T-P-T-P-A-P.

Ce composé peptidique est désigné par l'expression « E34Pc » lorsqu'il est sous forme de composé peptidique cyclisé en Cys9-Cys15.

Il permet de distinguer en zone d'endémie un hôte vacciné d'un hôte non vacciné mais infecté.
Le composé peptidique étudié dans la présente étude est le composé peptidique K-(palmitoyl)-E-D-E-H-K-G-K-Y-C-R-L-G-N-D-C-R-T-T-E-P-T-T-T-A-T-P-R-G-T-P-T-P-A-P.

Les composés peptidiques ont été préparés à 1mM dans une solution de 10%DMSO/PBS 1X (sans Mg/Ca) avec des fioles contenant 1mg de chaque composé peptidique.
Les tableaux 10, 11 et 12 représentent les résultats des études physico-chimiques des différents composés peptidiques cités ci-dessus.

**Tableau 10 : Poids moléculaires et équivalence à 1mM (millimole/L) des composés peptidiques retenus.**

| Type épitopes | Nom composés peptidiques ou *Liaison super type HLA* | Séquences composés peptidiques | Nb a.a | P.M g/mol | [mg/mL] équivalent **1mM** | [mM] équivalent 1mg/mL |
|---|---|---|---|---|---|---|
| **HLA Classe I** | SEQ ID N°11 | TPEQRTNTLTVELGK KWIG | **19** | 2171,48 | 2,171 | 4,61 x10⁻⁴ |
| | *B07* | *TPEQRTNTL* | | *1059,14* | | |
| | *B08* | *ELGKKWIG* | | *930,11* | | |
| | SEQ ID N°12 | TLPEMPVGVPEMPAG VDY | **18** | 1902,2 | 1,902 | 5,26 x10⁻⁴ |
| | *A02* | *TLPEMPVGV* | | *942,14* | | |
| | *A01* | *PEMPAGVDY* | | *978,08* | | |
| | SEQ ID N°13 | ARGREGYFLARGARG REGYEGYFVTDEK | **28** | 3211,5 | 3,212 | 3,11 x10⁻⁴ |
| | *B2* 7 | *ARGREGYFL* | | *1068,20* | | |
| | *B44 (B18) & B27* | *ARGARGREGY* | | *1092,18* | | |
| | *A03* (A11) | *EGYFVTDEK* | | *1087,15* | | |
| **HLA Classe II** | SEQ ID N°9 | TNTLAVLQAFGRAIP ELGKKW | **21** | 2313,73 | 2,314 | 4,32 x10⁻⁴ |
| | SEQ ID N°10 | EGYFVTDEKTGLVYR DGGVAAASSG | **25** | 2549,73 | 2,550 | 3,92 x10⁻⁴ |
| **B** | **E-34** | EDEHKGKYCRLGNDC RTTEPTTTATPRGTP TPAP | **34** | 3702,05 | 3,702 | 2,70 x10⁻⁴ |

**Tableau 11 : Composés peptidiques non Palmitoylés.**

| Type épitopes | Nom composés peptidiques | Séquences composés peptidiques | P.M g/mol | DMSO Volume mL | PBS 1X Volume mL | Volume final (mL) |
|---|---|---|---|---|---|---|
| **HLA Classe I** | SEQ ID N°11 | TPEQRTNTLTVELGKKWIG | 2171.4 8 | 0.046 | 0.415 | 0.461 |
| | SEQ ID N°12 | TLPEMPVGVPEMPAGVDY | 1902.2 | 0.053 | 0.473 | 0.526 |
| | SEQ ID N°13 | ARGREGYFLARGARGREGYEGY FVTDEK | 3211.5 | 0.031 | 0.280 | 0.311 |
| **HLA Classe II** | SEQ ID N° 9 | TNTLAVLQAFGRAIPELGKKW | 2313.73 | 0.043 | 0.389 | 0.432 |
| | SEQ ID N°10 | EGYFVTDEKTGLVYRDGGVAAA SSG | 2549.73 | 0.039 | 0.353 | 0.392 |

**Tableau 12 : Composés peptidiques palmitoylés. (Queue Palmitoyl PM= 366,64)**

| Type épitopes | Nom composés peptidiques | Séquences composés peptidiques palmitoylés | P.M g/mol | DMSO Volume mL | PBS 1X Volume mL | Volume final (mL) |
|---|---|---|---|---|---|---|
| **HLA Classe I** | **K-SEQ ID N°11** | K-(Pal)- TPEQRTNTLTVELGKKW IG | **2538,12** | 0,039 | 0,355 | 0,394 |
| | **K-SEQ ID N°12** | K-(Pal)- TLPEMPVGVPEMPAGVD Y | **2268,84** | 0,044 | 0,397 | 0,441 |
| | **K-SEQ ID N°13** | K-(Pal)- ARGREGYFLARGARGRE GYEGYFVTDEK | **3578,14** | 0,027 | 0,252 | 0,279 |
| **HLA Classe II** | **K-SEQ ID N°9** | K-(Pal)- TNTLAVLQAFGRAIPEL GKKW | **2680,37** | 0,037 | 0,336 | 0,373 |
| | **K-SEQ ID N°10** | K-(Pal)- EGYFVTDEKTGLVYRDG GVAAASSG | **2916,37** | 0,034 | 0,309 | 0,343 |
| **B** | **E-34-PC** | K-(Pal)- EDEHKGKYCRLGNDCRT TEPTTTATPRGTPTPAP | **4066,69** | 0,025 | 0,221 | 0,246 |

Ces résultats indiquent les propriétés biochimiques des peptides et permettent de préparer des solutions mères de peptides à 1mM en vue de tester, après dilution, leur efficacité chez le chien et sur cellules humaines comme illustré aux exemples 7 et 8 ci-dessous.

### Exemple 7 : Etude de l'état immunitaire des chiens avant et après vaccination

Les composés peptidiques SEQ ID N°9 à 13 de l'invention sont mélangés au composé peptidique de synthèse non natif E34Pc, SEQ ID N°14, qui permet de distinguer en zone d'endémie un hôte vacciné d'un hôte non vacciné mais infecté. Ce composé peptidique E34Pc est associé à un adjuvant induisant de préférence une réponse à médiation cellulaire tel que par exemple du QA21, de la saponine, de la Quil-A ou tout autre dérivé de ceux-ci connu de l'homme de métier, tel que le QS-21.

De manière préférée, les séquences peptidiques sont associées dans un rapport SEQ ID N°9 / SEQ ID N°10 / SEQ ID N°11 / SEQ ID N°12 / SEQ ID N°13 / SEQ ID N°14: 20 / 20 / 20 /15 / 25 / 10.

Les composés peptidiques selon l'invention peuvent être rendus immunogéniques par liaison à des porteurs ou tout autre dérivé (grosse molécule type KLH, lipopeptides type palmitoyl ou dérivés) et sont administrés aux candidats en présence d'un adjuvant et de préférence au QA21.

Afin de connaître la capacité du candidat vaccinal à induire une immunité protectrice, les réponses immunitaires humorale et cellulaire des compositions comprenant :
- les cinq composés peptidiques SEQ ID N°9 à SEQ ID N°13 ou
- les six composés peptidiques SEQ ID N°9 à SEQ ID N°14 lorsque le composé peptidique E34Pc est également présent, sont évaluées dans les compositions, chez les chiens immunisés (vaccinés) comparativement à des chiens placebos.

La composition de peptides est testée sur 38 chiens de race « Beagle » présentant une sérologie leishmaniose négative, une parasitologie négative, une absence de réponse cellulaire à Leishmania (INF-γ et granzyme B) et une absence d'IgG2 spécifiques du composé peptidique E34Pc, répartis en 10 groupes (0 à 9).

Dans l'établissement des groupes de chiens, il est notamment prévu de comparer les compositions selon l'invention à une combinaison des trois composés peptidiques décrits dans les documents brevets FR2829767 et FR2932802. Il s'agit des trois composés peptidiques suivants :
- A16E: A-A-R-C-A-R-L-R-E-G-Y-S-L-T-D-E
- A16G: A-A-S-S-T-P-S-P-G-S-G-C-E-V-D-G; et
- E34P : E-D-E-H-K-G-K-Y-C-R-L-G-N-D-C-R-T-T-E-P-T-T-T-A-T-P-R-G-T-P-T-P-A-P.
Ces trois peptides, sous forme palmitoylée sont associés dans un rapport 10/25/25 pour KE34P/KA17E/KA17G.

Cette combinaison constitue le groupe de référence, qui est comparé aux composés peptidiques de classe I de SEQ ID N° 11 à 13, et aux composés peptidiques de classe II de SEQ ID N° 9 à 10.

Le but de la présente étude est également d'évaluer un effet dose de la concentration des divers composés peptidiques de la composition vaccinale.

Les 38 chiens ont vécu dans le Nord de la France (Auxerre), zone non endémique, et ont été rapatriés dans le Sud Est de la France, 4 semaines avant la vaccination en période hivernale (période ne présentant pas de vecteurs phlébotomes à Leishmania). Parmi les 10 groupes, un groupe témoin placébo de 2 chiens est prévu, il s'agit du groupe 0. Les 9 autres groupes comprennent chacun 4 chiens recevant les divers composés peptidiques aux concentrations indiquées dans le tableau 13 ci-dessous. Les différents mélanges peptidiques sont formulés avec de l'adjuvant QA21, du Tween 80 et du TMS et lyophilisés.

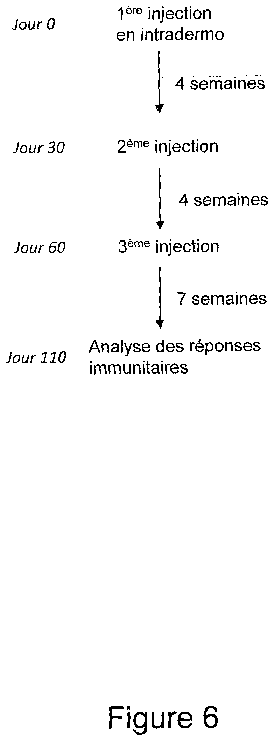

Le schéma d'injection est illustré à la figure 6. Les doses injectées sont, quant à elles, décrites dans le tableau 13 ci-dessous. Les valeurs indiquées dans ce tableau 13 correspondent aux quantités de peptides injectés en µg pour 200 µl de composition injectée par voie intradermique. Les groupes identifiés par une étoile dans le tableau 13 sont des groupes de 4 chiens. Les composés peptidiques notés « K » sont des composés peptidiques palmitoylés. Par exemple le composé peptidique « E34Pc palmitoylés est noté « KE34Pc ».

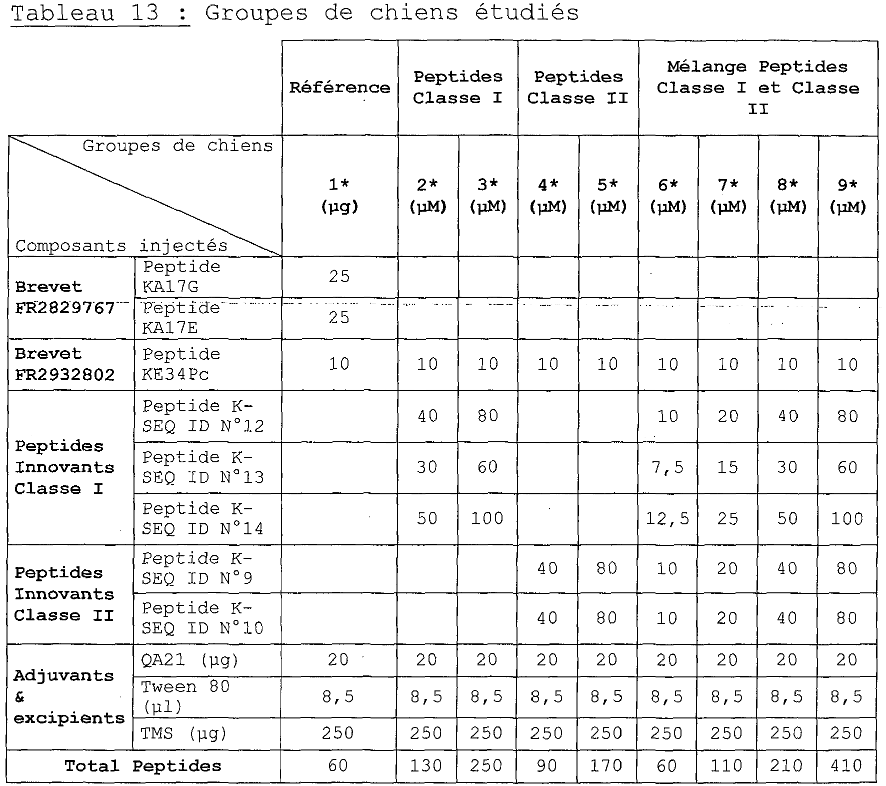

Un suivi clinique des 38 chiens est effectué toutes les 2 semaines.

Les analyses sont effectuées avant toute injection et 7 semaines après la 3^{ème} injection.

Dans un premier temps une recherche est effectuée dans les sérums des anticorps IgG2 spécifiques du composé peptidique E34Pc. Ces immunoglobulines de type IgG2 de chiens spécifiques du composé peptidique E34Pc sont détectées par méthode ELISA selon la méthode de microtitration de Kveider et al. (J. Immunol. 1987, 138-299) en utilisant un conjugué anti-IgG2. Pour cette méthode le composé peptidique est biotinylé avant coating sur microplaques selon la méthode décrite dans la demande de brevet FR2932802. Dans un second temps, la capacité des cellules mononuclées du sang périphérique (PBMC) à proliférer après 5 jours de stimulation par divers antigènes issus des candidats vaccins est évaluée et un dosage de la cytokine IFNγ (réponse Th1) est réalisé. Ces données sont obtenues par l'intermédiaire d'expériences ex *vivo* chez les chiens et permettent d'évaluer la réponse cellulaire.

La capacité des cellules mononuclées du sang périphérique (PBMC) à proliférer après 5 jours de stimulation par divers antigènes issus des candidats vaccins est évaluée par une technique ELISA (méthode alternative à la radioactivité).

Le dosage de la cytokine IFNγ se fait également par un test ELISA dans les surnageants des cellules stimulées et permet de déterminer la capacité des cellules à entrer dans une voie TH-1 après stimulation.

L'efficacité des candidats vaccins est déterminée ex *vivo* par un test d'activité leishmanicide. Ce test a pour but d'évaluer le type et le niveau d'intensité de la réponse immune à médiation cellulaire induite par l'administration d'un vaccin chez le chien, en étudiant la capacité des macrophages, mis en contact avec des lymphocytes autologues, à éliminer les parasites intracellulaires de formes amastigotes.

Enfin, un dosage de la sérine protéase Granzyme B se fait dans les surnageants des cellules stimulées afin d'évaluer la réponse cytotoxique lymphocytaire spécifique et de mettre en évidence la production de Granzyme B spécifique des lymphocytes T cytotoxiques. Ce dosage est réalisé par l'étude du niveau d'expression des transcrits par RT-q-PCR.

Le tableau 14 ci-dessous donne les résultats de ces divers tests biologiques, et recense donc le nombre de chiens donnant une réponse immunitaire positive :

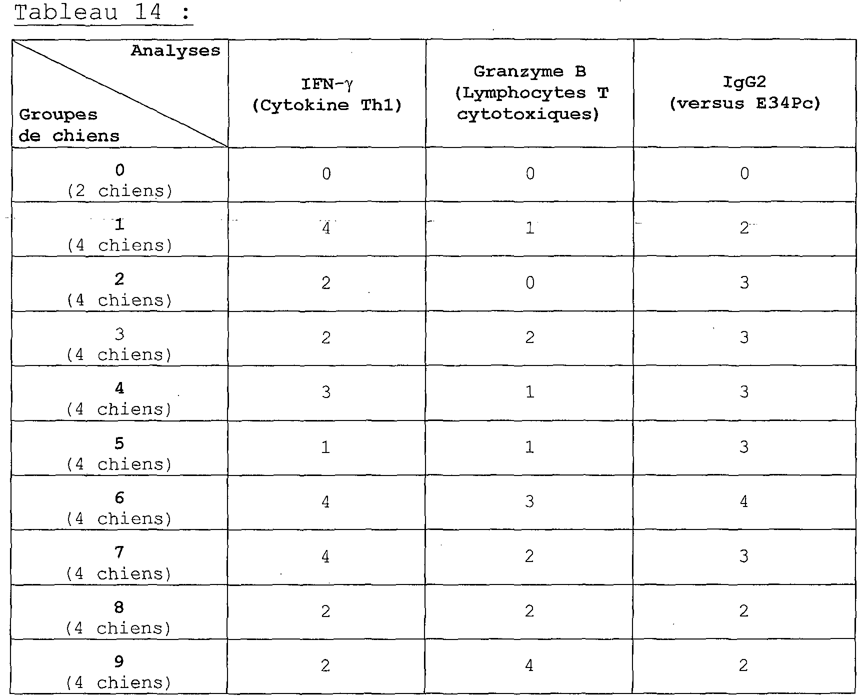

Comme cela est montré dans le tableau 14 ci-dessus, la combinaison des composés peptidiques de classe I et de classe II (groupes 6 à 9) donne de meilleurs résultats en terme de réponse immunitaire protectrice que les composés peptidiques de référence (groupe 1) et que les composés peptidiques de classe I et de classe II pris séparément (groupes 2, 3 et groupes 4, 5).

Parmi les groupes recevant la composition totale des composés peptidiques (Classe I, Classe II et E34Pc), les groupes 6 et 7 correspondants à de faibles concentrations en composés peptidiques donnent les meilleurs résultats. Il faut notamment noter les bons résultats du groupe 6 qui correspond à la plus basse concentration en composés peptidiques de la composition soit 60 µg de composés peptidiques par injection.

Il a ainsi été démontré que la composition agit par stimulation du système lymphocytaire de type Th1 avec production de lymphocytes T cytotoxiques et d'IgG2 spécifiques du composé peptidique E34Pc. L'intérêt de ces IgG2 ne repose pas seulement sur la distinction des chiens vaccinés/chiens infectés mais également sur leur capacité à inhiber la prolifération des formes amastigotes ou promastigotes de *Leishmania.*

### Exemple 8 : Efficacité ex vivo des composés peptidiques classe I et II de PSA à stimuler des cellules humaines et à produire des cytokines de type Th1

Les patients atteints de leishmaniose et guéris avec succès par la chimiothérapie sont généralement à l'abri de nouvelles infections à *Leishmania.* Cette résistance de l'hôte à l'infection leishmanienne est principalement médiée par des réponses immunes cellulaires spécifiques et notamment une lymphoprolifération des lymphocytes T et la production des cytokines Th1 comme l'interféron-gamma (IFN-γ) et interleukine (IL)-2 conduisant à l'activation des macrophages et entrainant la mort des parasites. Bien que des progrès significatifs aient été accomplis récemment pour comprendre les mécanismes de l'immunité chez l'Homme, aucun vaccin n'est actuellement disponible contre les leishmanioses humaines. Néanmoins, de nombreux efforts ont été réalisés pour identifier des antigènes de leishmanies capables d'induire une immunité protectrice. Les principaux travaux relatifs à la vaccination ont été et sont encore réalisés chez la souris. Ces modèles d'infection expérimentale sont très éloignés des hôtes naturels de l'infection, c'est-à-dire le chien et l'Homme, hôtes ayant un impact considérable en termes de santé vétérinaire et humaine. De plus, ces travaux ont concerné essentiellement la leishmaniose cutanée à *L. major* (principal modèle d'infection disponible chez la souris), pour laquelle les études ne sont pas directement transposables à la leishmaniose viscérale canine ou humaine.

Le Demandeur a donc mis au point une nouvelle approche qui consiste à évaluer la capacité des antigènes de vaccins candidats à stimuler les lymphocytes T du sang périphérique de patients guéris ou de sujet exposés asymptomatiques et immuns en mesurant *ex vivo* la production de cytokines Th1 et/ou Th2. L'efficacité de ces antigènes à déclencher notamment une réponse Th1 (libération d'IFN-γ par les lymphocytes T stimulés) et l'intensité de la réponse provoquée seront mis en corrélation avec la protection ou la guérison, Ce test *ex vivo* d'efficacité de candidats vaccins sur cellules humaines a été utilisé avec la PSA recombinante native à 10µM et le vaccin candidat peptidique (Pool A = combinaison des cinq composés peptidiques SEQ ID N°9 à 13) à 2 µM sur des cellules d'individus asymptomatiques immuns comparativement à des sujets naïfs.

Le statut des individus a été défini par un examen clinique mais aussi par des méthodes immunologiques (prolifération cellulaire contre des antigènes solubles totaux de leishmanies, dosage d'IFN-γ après stimulation cellulaire, et dosage des anticorps dirigés contre ces antigènes dans le plasma) et parasitologiques (recherche et quantification de parasites dans le sang par RT-Q-PCR). Les groupes d'individus ont donc été définis comme suit :
- Individus naïfs : aucun historique médical concernant une leishmaniose, absence de signes cliniques, prolifération cellulaire négative, absence de production d'IFNγ après stimulation cellulaire, aucune évidence de la présence d'anticorps dirigés contre les antigènes totaux de leishmanies et PCR négative ;
- Individus asymptomatiques : absence de signes cliniques, prolifération cellulaire positive et/ou production -d'IFN-γ après stimulation des cellules par les antigènes totaux de leishmanies, PCR positive ou négative.

Les résultats sont présentés dans le tableau 15 ci-dessous :

**Tableau 15 : Taux d'IFN- y secrétés par les cellules T du sang périphérique isolées d'individus asymptomatiques (asympt) et de sujets naïfs.**

| | | | | [IFN-Y pg/mL] | | | | | |
|---|---|---|---|---|---|---|---|---|---|
| | CBA standard | | | Milieu | Valeur Antigène - Valeur Milieu | | | | |
| Groupes d'individus | Top standard concentrat° pg/mL | Mean MFI top standard | SD | Milieu seul | PHA | TSLA Ldd8 | TSLA Ldi | nsLaPSA | Pool A 2µM |
| Asympt 1 | 5000 | 2712,9 | 922,39 | 68,48 | ND | 96,71 | 132,08 | 93,68 | 61,35 |
| Asympt 2 | 5000 | 2712,9 | 922,39 | 128,63 | ND | 292,07 | 94,5 | 217,67 | 331,44 |
| Asympt 3 | 5000 | 2712,9 | 922,39 | 0 | ND | 89,85 | 97,42 | 113,15 | 137,75 |
| Asympt 4 | 5000 | 1538,93 | 536,14 | 135,29 | 6812,34 | 29,88 | 352,35 | 250,62 | 175,03 |
| Asympt 5 | 5000 | 2712,9 | 922,39 | 115,04 | ND | 247,68 | 579,17 | 767,32 | 261,44 |
| | | | | | | | | | |
| Naïf 1 | 5000 | 2712,9 | 922,39 | 318,48 | ND | -111,33 | -189,85 | -223,07 | -200,18 |
| Naïf 2 | 5000 | 2146,99 | 712,46 | 164,18 | 2923,49 | -157,29 | -125,61 | -69,33 | -89,82 |
| Naïf 3 | 5000 | 2146,99 | 712,46 | 533,91 | 3424,42 | -432,27 | -356,45 | -446,28 | -416,12 |
| Naïf 4 | 5000 | 2146,99 | 712,46 | 614,13 | 3189,57 | -129,33 | 8,96 | -105,37 | -147,68 |
| Naïf 5 | 5000 | 2146,99 | 712,46 | 232,79 | 3245,22 | -128,11 | -155,78 | -8,84 | -137 |

| | | | | | | | | | |
|---|---|---|---|---|---|---|---|---|---|
| TSLA: antigènes totaux de leishmanie; nsLaPSA : protéine PSA recombinante ; Pool A : combinaison des composés peptidiques de synthèse de SEQ ID N°9 à 13. | | | | | | | | | |

Comme le montrent les résultats, seules les cellules d'individus asymptomatiques, comparativement à celles des sujets naïfs, sont susceptibles d'induire une réponse Th1 (stimulation des lymphocytes T avec production significative d'IFN-y) en présence de PSA recombinante ou du Pool A. Ce dernier représente donc un excellent candidat pour les vaccins prophylactiques et thérapeutiques contre les leishmanioses humaines.

## Revendications

1. Epitope présentant une séquence choisie parmi SEQ ID N°1 à SEQ ID N°10.

2. Epitope selon la revendication 1, présentant une séquence choisie parmi SEQ ID N°1, 2, 8, 9 ou 10.

3. Composé peptidique de 18 à 28 acides aminés comprenant 1, 2, 3 ou 4 des épitopes selon l'une des revendications 1 ou 2, éventuellement séparés par un espaceur peptidique comprenant 1 à 8 acides aminés.

4. Composé peptidique selon la revendication 3 choisi parmi SEQ ID N°9 à SEQ ID N°13.

5. Composition pharmaceutique, à usage vétérinaire ou humain comprenant au moins un épitope selon la revendication 1 ou 2, ou un composé peptidique selon l'une des revendications 3 ou 4.

6. Composition selon la revendication 5, pour son utilisation dans la vaccination prophylactique et thérapeutique dirigée contre les leishmanies tels que *Leishmania donovani, Leishmania infantum, Leishmania chagasi, Leishmania mexicana, Leishmania amazonensis, Leishmania venezuelensis, Leishmania tropica, Leishmania major, Leishmania aethiopica, Leishmania* (*Viannia) braziliensis, Leishmania* (*Viannia) guyanensis, Leishmania* (*Viannia) panamensis, Leishmania* (*Viannia) peruviana.*

7. Vaccin prophylactique et/ou thérapeutique dirigé contre une ou plusieurs des leishmanies choisies parmi *Leishmania donovani, Leishmania infantum, Leishmania chagasi, Leishmania mexicana, Leishmania amazonensis, Leishmania venezuelensis, Leishmania tropica, Leishmania major, Leishmania aethiopica, Leishmania* (*Viannia) braziliensis, Leishmania* (*Viannia) guyanensis, Leishmania* (*Viannia) panamensis, Leishmania* (*Viannia) peruviana ;*
comprenant au moins un épitope selon la revendication 1 ou 2, ou un composé peptidique selon l'une des revendications 3 ou 4.

8. Vaccin selon la revendication 7, **caractérisé en ce qu'**il comprend d'une part, au moins un composé peptidique choisi parmi SEQ ID N°9 et 10 ;
et d'autre part, au moins un composé peptidique multi-épitopiques choisi parmi les séquences SEQ ID N°11 à 13.

9. Vaccin selon la revendication 8, **caractérisé en ce qu'**il comprend d'une part, au moins un ou deux composés peptidiques choisis parmi SEQ ID N°9 et 10 ;
et d'autre part, au moins un, deux ou trois composés peptidiques choisis parmi les séquences SEQ ID N°11 à 13.

10. Vaccin selon la revendication 9, **caractérisé en ce qu'**il comprend, en combinaison, cinq composés peptidiques multiépitopiques choisis parmi SEQ ID N°9 à 13.

11. Vaccin selon l'une des revendications 6 à 9, **caractérisé en ce qu'**il comprend en outre un adjuvant choisi parmi les classes TLR3, TLR4, TLR5, TLR7, TLR8, TLR9, les saponines, les émulsions huile dans eau ou eau dans huile, les polysaccharides, les liposomes cationiques, les virosomes ou les poly-electrolytes.

12. Vaccin selon l'une des revendications 7 à 11, **caractérisé en ce qu'**il comprend en outre le composé peptidique E34P de SEQ ID N°14 suivante:
E-D-E-H-K-G-Y-C-RL-G-N-D-C-R-T-T-E-P-T-T-T-A-T-P-R-G-T-P-T-P-A-P, cyclisé par pont disulfure entre la cystéine en position 9 et la cystéine en position 15.

13. Vaccin selon l'une des revendication 7 à 12, **caractérisé en ce qu'**il comprend en outre un antigène capable de générer des anticorps spécifiques lors d'une immunisation.

14. Séquences nucléotidiques codant pour l'épitope ou le composé peptidique tel que défini dans l'une des revendications 1 à 4.

15. Vecteur d'expression comprenant au moins une séquence nucléotidique telle que définie à la revendication 14, ainsi que les moyens nécessaires à leur expression.

16. Réactif de diagnostic comprenant un composé peptidique selon l'une des revendications 1 à 4.

## Patentansprüche

1. Epitop, eine Sequenz aufweisend ausgewählt aus SEQ ID NO 1 bis SEQ ID NO 10.

2. Epitop nach Anspruch 1, eine Sequenz aufweisend ausgewählt aus SEQ ID NO 1, 2, 8, 9 oder 10.

3. Peptid-Verbundstoff aus 18 bis 28 Aminosäuren, umfassend 1, 2, 3 oder 4 der Epitope nach einem der Ansprüche 1 oder 2, gegebenenfalls getrennt durch einen 1 bis 8 Aminosäuren umfassenden Peptid-Abstandhalter.

4. Peptid-Verbundstoff nach Anspruch 3, ausgewählt aus SEQ ID NO 9 bis SEQ ID NO 13.

5. Pharmazeutische Zusammensetzung zur veterinär- oder humanmedizinischen Verwendung, umfassend wenigstens ein Epitop gemäß Anspruch 1 oder 2 oder einen Peptid-Verbundstoff nach einem der Ansprüche 3 oder 4.

6. Zusammensetzung nach Anspruch 5 zur Verwendung bei der prophylaktischen und therapeutischen Impfung gegen Leishmanien wie *Leishmania donovani, Leishmania infantum, Leishmania chagasi, Leishmania mexicana, Leishmania amazonensis, Leishmania venezulensis, Leishmania tropica, Leishmania major, Leishmania aethiopica, Leishmania* (*Viannia) braziliensis, Leishmania* (*Viannia) guyanensis, Leishmania* (*Viannia) panamensis, Leishmania* (*Viannia) peruviana.*

7. Phrophylaktischer und/oder therapeutischer Impfstoff gegen eine oder mehrere Leishmanien ausgewählt aus *Leishmania donovani, Leishmania infantum, Leishmania chagasi, Leishmania mexicana, Leishmania amazonensis, Leishmania venezulensis, Leishmania tropica, Leishmania major, Leishmania aethiopica, Leishmania* (*Viannia) braziliensis, Leishmania* (*Viannia) guyanensis, Leishmania* (*Viannia) panamensis, Leishmania* (*Viannia) peruviana;* umfassend wenigstens ein Epitop gemäß Anspruch 1 oder 2 oder einen Peptid-Verbundstoff nach einem der Ansprüche 3 oder 4.

8. Impfstoff nach Anspruch 7, **dadurch gekennzeichnet, dass** er einerseits wenigstens eine Peptid-Zusammensetzung umfasst, ausgewählt aus SEQ ID NO 9 und 10;
und andererseits wenigstens einen multi-epitopischen Peptid-Verbundstoff, ausgewählt aus den Sequenzen SEQ ID NO 11 bis 13.

9. Impfstoff nach Anspruch 8, **dadurch gekennzeichnet, dass** er einerseits wenigstens einen oder zwei Peptid-Verbundstoffe ausgewählt aus SEQ ID N°9 und 10 umfasst; und andererseits wenigstens einen, zwei oder drei Peptid-Verbundstoffe, ausgewählt aus den Sequenzen SEQ ID NO 11 bis 13.

10. Impfstoff nach Anspruch 9, **dadurch gekennzeichnet, dass** er in Kombination fünf multieptitope Peptid-Zusammensetzungen umfasst, ausgewählt aus SEQ ID NO 9 bis 13.

11. Impfstoff nach einem der Ansprüche 6 bis 9, **dadurch gekennzeichnet, dass** er weiter einen Hilfsstoff umfasst ausgewählt aus den Klassen TLR3, TLR4, TLR5, TLR7, TLR8, TLR9, Saponinen, Öl-in-Wasser- oder Wasser-in-Öl-Emulsionen, Polysaccariden, kationischen Liposomen, Virosomen oder Poly-Elektrolyten.

12. Impfstoff nach einem der Ansprüche 7 bis 11, **dadurch gekennzeichnet, dass** er weiter den Peptid-Verbundstoff E34P der folgenden SEQ ID NO 14 umfasst:
E-D-E-H-K-G-Y-C-RL-G-N-D-C-R-T-T-E-P-T-T-T-A-T-P-R-G-T-P-T-P-A-P, cyclisiert durch eine Disulfur-Brücke zwischen dem Cystein in Position 9 und dem Cystein in Position 15.

13. Impfstoff nach einem der Ansprüche 7 bis 12, **dadurch gekennzeichnet, dass** er weiter einen Antikörper umfasst, fähig, bei einer Immunisierung spezifische Antikörper zu erzeugen.

14. Codierende Nukleotidsequenzen für das Epitop oder den Peptid-Verbundstoff wie in einem der Ansprüche 1 bis 4 definiert.

15. Expressionsvektor, umfassend wenigstens eine Nukleotidsequenz wie in Anspruch 14 definiert sowie die für deren Expression erforderlichen Mittel.

16. Diagnostisches Reagens, umfassend einen Peptid-Verbundstoff nach einem der Ansprüche 1 bis 4.

## Claims

1. Epitope having a sequence selected from SEQ ID NO. 1 to SEQ ID NO 10.

2. Epitope according to claim 1, having a sequence selected from SEQ ID NO. 1, 2, 8, 9 or 10.

3. Peptide compound of 18 to 28 amino acids comprising 1, 2, 3 or 4 of the epitopes according to one of claims 1 or 2, optionally separated by a peptide spacer comprising 1 to 8 amino acids.

4. Peptide compound according to claim 3 selected from SEQ ID NO. 9 to SEQ ID NO. 13.

5. Pharmaceutical composition, for veterinary or human use comprising at least one epitope according to claim 1 or 2, or a peptide compound according to one of claims 3 or 4.

6. Composition according to claim 5, for use in preventive and therapeutic vaccination targeted against Leishmania such as *Leishmania donovani, Leishmania infantum, Leishmania chagasi, Leishmania mexicana, Leishmania amazonensis, Leishmania venezuelensis, Leishmania tropica, Leishmania major, Leishmania aethiopica, Leishmania* (*Viannia) braziliensis, Leishmania* (*Viannia) guyanensis, Leishmania* (*Viannia) panamensis, Leishmania* (*Viannia) peruviana.*

7. Preventive and/or therapeutic vaccine targeted against one or more of the Leishmania selected from *Leishmania donovani, Leishmania infantum, Leishmania chagasi, Leishmania mexicana, Leishmania amazonensis, Leishmania venezuelensis, Leishmania tropica, Leishmania major, Leishmania aethiopica, Leishmania* (*Viannia) braziliensis, Leishmania* (*Viannia) guyanensis, Leishmania* (*Viannia) panamensis, Leishmania* (*Viannia) peruviana;*
comprising at least one epitope according to claim 1 or 2, or a peptide compound according to one of claims 3 or 4.

8. Vaccine according to claim 7, **characterised in that** it comprises, on one hand, at least one peptide compound selected from SEQ ID NO. 9 and 10;
and, on the other, at least one multi-epitope peptide compound selected from the sequences SEQ ID NO. 11 to 13.

9. Vaccine according to claim 8, **characterised in that** it comprises, on one hand, at least one or two peptide compounds selected from SEQ ID NO. 9 and 10;
and, on the other, at least one, two or three peptide compounds selected from the sequences SEQ ID NO. 11 to 13.

10. Vaccine according to claim 9, **characterised in that** it comprises, in combination, five multi-epitope peptide compounds selected from SEQ ID NO. 9 to 13.

11. Vaccine according to one of claims 6 to 9, **characterised in that** it further comprises an adjuvant selected from the classes TLR3, TLR4, TLR5, TLR7, TLR8, TLR9, saponins, oil-in-water or water-in-oil emulsions, polysaccharides, cationic liposomes, virosomes or poly-electrolytes.

12. Vaccine according to one of claims 7 to 11, **characterised in that** it further comprises the peptide compound E34P of following SEQ ID NO. 14:
E-D-E-H-K-G-Y-C-RL-G-N-D-C-R-T-T-E-P-T-T-T-A-T-P-R-G-T-P-T-P-A-P, cyclised by disulphide bridge between cysteine in position 9 and cysteine in position 15.

13. Vaccine according to one of claims 7 to 12, **characterised in that** it further comprises an antigen capable of generating specific antibodies during an immunisation.

14. Nucleotide sequences coding for the epitope of the peptide compound as defined in one of claims 1 to 4.

15. Expression vector comprising at least one nucleotide sequence as defined in claim 14, as well as the means required for the expression thereof.

16. Diagnostic reagent comprising a peptide compound according to one of claims 1 to 4.
